# EUROPÄISCHE PATENTSCHRIFT

(11) **EP 2 496 152 B1**
(45) Veröffentlichungstag und Bekanntmachung des Hinweises auf die Patenterteilung: **11.01.2017**
(21) Anmeldenummer: 10778565.1
(22) Anmeldetag: 05.11.2010
(51) Int. Cl.: A61B 17/3207, A61B 17/22, A61B 17/221, A61B 17/00, A61B 17/30

(54) **MEDIZINISCHE VORRICHTUNG ZUM REKANALISIEREN VON KÖRPERHOHLRÄUMEN, SET UMFASSEND EINE DERARTIGE VORRICHTUNG UND VERFAHREN ZUR HERSTELLUNG EINER MEDIZINISCHEN VORRICHTUNG**
DEVICE FOR RECANALISATION OF BODY LUMEN, SET COMPRISING SAID DEVICE AND METHODS FOR MANUFACTURE OF MEDICAL DEVICE
DISPOSITIF POUR RECANALISATION D'UN ORGANE TUBULAIRE, KIT COMPRENANT UN TEL DISPOSITIF ET MÉTHODE POUR SA FABRICATION

(30) Priorität: 05.11.2009 DE 102009052002
(43) Veröffentlichungstag der Anmeldung: 12.09.2012
(73) Patentinhaber: Acandis GmbH & Co. KG, 76327 Pfinztal (DE)
(72) Erfinder: CATTANEO, Giorgio, 76199 Karlsruhe (DE)
(74) Vertreter: Kilchert, Jochen
(86) Internationale Anmeldenummer: PCT/EP2010/006758
(87) Internationale Veröffentlichungsnummer: WO 2011/054531

(56) Entgegenhaltungen:
- WO-A1-96/13295
- WO-A1-96/26682
- WO-A2-02/28291
- WO-A2-2004/037178
- WO-A2-2007/092506
- US-A1- 2004 143 209

## Beschreibung

Die Erfindung betrifft eine medizinische Vorrichtung zum Rekanalisieren von Körperhohlräumen gemäß dem Oberbegriff des Anspruchs 1 sowie ein Set umfassend eine derartige medizinische Vorrichtung.

Die Bildung von Konkrementen in Körperhohlräumen, insbesondere von Thromben in Blutgefäßen, kann zu einem Verschluss des Körperhohlraums führen. Bei einem Verschluss von Blutgefäßen kommt es zu einer Unterversorgung des nachfolgenden Gewebes mit Nährstoffen und Sauerstoff und in der Folge zu gesundheitlichen Beeinträchtigungen für den Patienten. Um dem entgegenzuwirken, besteht das medizinische Ziel, die Blockierung des Körperhohlraums insbesondere des Blutgefäßes, zu lösen, also das Gefäß zu rekanalisieren.

Eine technische Möglichkeit zur Lösung dieses medizinischen Problems besteht darin, das Blutgefäß im Bereich des Thrombus aufzuweiten, um dadurch eine Strömungspassage am Thrombus vorbei zu eröffnen. Geeignete Vorrichtungen umfassen meist ein stentartiges Gebilde, das durch einen Katheter an den Behandlungsort geführt wird. Der Stent wird im Bereich des Thrombus freigesetzt, wobei eine Radialkraft, die der Stent auf den Thrombus und das Blutgefäß ausübt, bewirkt, dass der Thrombus an die Gefäßwand gedrückt wird. Durch die Öffnungen in der Stentwandung kann das Blut hindurchströmen, so dass eine Rekanalisation erfolgt. Gleichzeitig zur mechanischen Aufweitung des Blutgefäßes wird eine medikamentöse Behandlung durchgeführt, wobei antithrombogene Wirkstoffe in die Blutbahn injiziert werden, die im Bereich des Thrombus ihre Wirkung entfalten. Dieser Vorgang wird als medikamentöse Lyse bezeichnet. Bei der medikamentösen Lyse besteht jedoch die Gefahr, dass der Thrombus nur teilweise aufgelöst wird. In der Folge können unvollständig gelöste Thrombuspartikel durch die Blutströmung mitgerissen werden und weitere Gefäßverschlüsse verursachen.

Für die weitere Behandlung bleiben in diesem Fall zwei Möglichkeiten. Einerseits kann der Stent bzw. das stentartige Gebilde vollständig im Gefäß freigesetzt werden, um sicherzustellen, dass die verbleibenden Thrombusbestandteile nicht durch die Blutströmung weitergeschwemmt werden. In der Folge ist eine langfristige medikamentöse Behandlung des Patienten mit gerinnungshemmenden Mitteln erforderlich, um die Bildung weiterer Blutgerinnsel im Bereich des Stents zu vermeiden. Durch die Verabreichung von gerinnungshemmenden Mitteln erhöht sich allerdings das Blutungsrisiko. Überdies sind mit einem dauerimplantierten Stent weitere Risiken verbunden, beispielsweise die Gefahr der In-Stent-Thrombose sowie entzündliche Reaktionen der Gefäßwand. Andererseits besteht bei verbleibenden Thrombenbestandteilen nach der medikamentösen Behandlung die Möglichkeit, den Thrombus mit Hilfe des stentartigen Gebildes zu entfernen, wobei das stentartige Gebilde aus dem Blutgefäß herausgezogen wird, sobald es sich im Bereich des Stents aufgespannt hat. Dabei wird riskiert, dass sich Thrombusbestandteile beim Transport ablösen, insbesondere beim Übergang in Gefäße mit größerem Querschnittsdurchmesser. Die abgelösten Thrombusbestandteile können in stromabwärts gelegene, kleinere Gefäße geschwemmt werden und zu weiteren Gefäßverschlüssen führen.

Eine weitere medizinische Möglichkeit zur Wiederherstellung der Gewebedurchblutung stromabwärts eines Gefäßverschlusses besteht in der vollständigen Entfernung des Thrombus. Eine dazu geeignete Vorrichtung ist beispielsweise aus der Druckschrift WO 2008/088920 A2 bekannt. Die bekannte Vorrichtung umfasst einen Katheter, in dem ein Führungsdraht längsverschieblich angeordnet ist. Der Führungsdraht umfasst eine expandierbare Spitze, die im expandierten Zustand eine spiralförmige Struktur aufweist. Zur Entfernung eines Thrombus wird der Katheter durch den Thrombus hindurchgeschoben, wobei beim Zurückziehen des Katheters die Spitze des Führungsdrahtes freigesetzt wird. Die Spitze nimmt die spiralförmige, korkenzieherartige Struktur an und verhakt sich mit dem Thrombus, so dass dieser durch Zurückziehen des Führungsdrahtes aus dem Blutgefäß entfernt werden kann. Um zu verhindern, dass sich Bestandteile des Thrombus ablösen und zu weiteren Gefäßverschlüssen führen, ist es notwendig, den Führungsdraht langsam und vorsichtig aus dem Blutgefäß zu ziehen. Diese Notwendigkeit verhindert jedoch eine schnelle Entfernung des Thrombus und somit eine schnelle Rekanalisation des Blutgefäßes. Das medizinische Ziel, eine möglichst schnelle Wiederherstellung des Blutflusses zu erreichen, wird dadurch nicht erreicht.

Die Druckschrift WO 2004/037178 A2 beschreibt eine medizinische Vorrichtung zur Entfernung und Auflösung eines Blutgerinnsels. Die bekannte medizinische Vorrichtung umfasst einen Katheter zum Freisetzen von thrombolytischen Substanzen. Der Katheter umfasst ein proximales Ende und eine distale, nicht lineare Region. Das thrombolytische Mittel wird über Öffnungen in der nicht linearen Region in das Gerinnsel eingebracht. Die nicht lineare Region kann von einem komprimierten in einen expandierten Zustand übergehen. Im komprimierten Zustand liegt die nicht lineare Region gestreckt vor, wogegen die nicht lineare Region im expandierten Zustand eine spiralförmige Windung bildet. Der Katheter weist, auch in der nicht linearen Region, einen konstanten Querschnittsdurchmesser auf. Der konstante Durchmesser legt die Größe der Arterien fest, in welche der Katheter eingeführt werden kann. Dies hat den Nachteil, dass der bekannte Katheter nicht flexibel für verschiedene Arteriengrößen verwendet werden kann.

Weitere medizinische Vorrichtungen zur Behandlung von Konkrementen in Körperhohlräumen sind beispielhaft in den Druckschriften WO 2007/092506 A2, WO 02/28291 A2, US 2004/143209 A1, WO 96/13295 A1 und WO 96/26682 A1 offenbart.

Die Aufgabe der Erfindung besteht darin, eine Vorrichtung anzugeben, die einerseits eine schnelle und zuverlässige Rekanalisierung eines Körperhohlraums und andererseits eine sichere Entfernung eines Konkrements aus einem Körperhohlraum ermöglicht. Ferner besteht die Aufgabe der Erfindung darin, ein Set umfassend eine derartige Vorrichtung anzugeben bzw. ein Verfahren zur Herstellung einer derartigen Vorrichtung anzugeben.

Erfindungsgemäß wird diese Aufgabe im Hinblick auf die medizinische Vorrichtung durch den Gegenstand des Patentanspruchs 1 im Hinblick auf das Set durch den Gegenstand des Patentanspruchs 14 gelöst und im Hinblick auf das Verfahren durch den Gegenstand des Anspruchs 15 gelöst.

Die Erfindung beruht auf dem Gedanken, eine medizinische Vorrichtung zum Rekanalisieren von Körperhohlräumen mit einem in den Körperhohlraum einführbaren Rekanalisationselement anzugeben. Das Rekanalisationselement ist von einem komprimierten Zustand in einen expandierten Zustand überführbar. Das Rekanalisationselement weist im expandierten Zustand einen rohrförmigen Hohlkörper auf derart, dass der Körperhohlraum rekanalisierbar ist. Die Außenkontur des Rekanalisationselements bildet, insbesondere im expandierten Zustand, wenigstens eine spiralförmige Windung.

Die erfindungsgemäße Lösung kombiniert also die Vorteile der aus dem Stand der Technik bekannten Systeme in neuartiger Weise, indem einerseits eine schnelle und zuverlässige Rekanalisation und andererseits eine sichere Entfernung des Konkrements erreicht wird. Dazu weist die erfindungsgemäße Vorrichtung einen äußerst einfachen und sicher handhabbaren Aufbau auf, nämlich den spiralförmig gewundenen, rohrförmigen Hohlkörper. Mit anderen Worten umfasst das Rekanalisationselement einen rohrförmigen Hohlkörper, der im Wesentlichen rotationssymmetrisch ausgebildet ist, wobei die Rotationsachse bzw. die Mittellängsachse des Hohlkörpers im expandierten Zustand spiralförmig gewunden ist. Das Rekanalisationselement kann also eine im Wesentlichen korkenzieherartige Außenstruktur aufweisen, wobei die Korkenzieherwindungen durch den rohrförmigen Hohlkörper gebildet sind. Die rotationssymmetrische Konfiguration des Hohlkörpers oder der durch den Hohlkörper gebildeten spiralförmigen Windung schließt nicht aus, dass der Außendurchmesser des Hohlkörpers oder der spiralförmigen Windung variabel ist.

Bei einer bevorzugten Ausführungsform der erfindungsgemäßen Vorrichtung ist der Hohlkörper durch eine rohrförmige Wandung begrenzt, die zumindest in einem proximalen Abschnitt eine Öffnung aufweist. Durch die Öffnung in einem proximalen Abschnitt der Hohlkörperwandung ist sichergestellt, dass der Hohlkörper, der aufgrund seiner rohrförmigen Struktur zumindest an einem distalen Ende des Rekanalisationselements eine axiale Öffnung aufweisen kann, vollständig durchströmbar ist. Bei Verwendung des Rekanalisationselements in einem Blutgefäß wird also durch die Öffnung im proximalen Abschnitt der Wandung sichergestellt, dass die Blutströmung in den Hohlkörper eintreten kann, so dass die Rekanalisationswirkung des Hohlkörpers erreicht ist. Alternativ oder zusätzlich kann in einem distalen Abschnitt des Hohlkörpers eine Öffnung vorgesehen sein.

Generell bildet die Wandung eine in Umfangsrichtung gekrümmte Begrenzungsfläche oder Begrenzungshülle, insbesondere eine im Umfangsrichtung gekrümmte, kontinuierliche bzw. gewebeartige Begrenzungsfläche, die die Außenkontur des Hohlkörpers bestimmt. Die Wandung bildet also eine im Wesentlichen flächige Außenkontur des Hohlkörpers.

Die Wandung kann zumindest abschnittsweise eine perforierte Struktur aufweisen. Die perforierte Struktur gewährleistet eine sichere Rekanalisation, wobei im Gebrauch weitere Strömungskanäle, die einen Kanal um den Thrombus bilden, eröffnet werden. Beispielsweise kann - in Abhängigkeit der gewählten Steigung der spiralförmigen Windung - durch den Hohlkörper mit der perforierten Wandungsstruktur eine Strömungspassage bereitgestellt werden, die vorwiegend entlang der Gefäßwand verläuft. Das bedeutet, dass ein größerer Anteil der Rekanalisationsströmung in axialer Richtung bezüglich des Hohlkörpers durch die perforierte Struktur hindurchtritt. Ein kleinerer Anteil umströmt das Konkrement in Umfangsrichtung durch den spiralförmigen Hohlkanal, den der Hohlkörper bildet. Dazu ist ein besonders flacher Steigungswinkel zweckmäßig. Bei einem steileren Steigungswinkel kann der Hohlkörper hingegen eine Strömungspassage bilden, die sich in Bezug auf das Körperhohlgefäß nicht vorwiegend in axialer Richtung erstreckt, sondern primär oder nahezu ausschließlich eine Umfangskomponente aufweist. Es ist auch möglich, dass sich die axiale Strömungspassage entlang der Gefäßwand und die sich über den Umfang erstreckende Strömungspassage überlagern. Die perforierte Struktur bietet den weiteren Vorteil, dass eine zusätzliche medikamentöse Behandlung, beispielsweise eine Thrombolyse, effizienter durchführbar ist, da über die perforierte Struktur ein im Wesentlichen großflächiger Zugang, also eine relativ große Angriffsfläche, zum Konkrement bereitgestellt wird.

In diesem Zusammenhang wird darauf hingewiesen, dass sich die Anzahl der fadenförmigen Elemente auf eine Querschnittsebene des Hohlkörpers bezieht. Dabei ist nicht ausgeschlossen, dass das Rekanalisationselement beispielsweise insgesamt aus der Hälfte der angegebenen fadenförmigen Elemente hergestellt ist, die jeweils an einem Ende des Hohlkörpers umgelenkt und zurückgeführt sind.

Bei einer bevorzugten Ausführungsform umfasst die geflochtene Gitterstruktur fadenförmige Elemente, die an einem distalen Ende der Gitterstruktur geschlossene Schlaufen bilden. Durch die Schlaufen wird ein atraumatisches Ende der Gitterstruktur bereitgestellt, wodurch Verletzungen der Gefäßwand vermieden werden.

Vorzugsweise umfasst die Wandung, insbesondere die perforierte Struktur, ein lasergeschnittenes oder in Dünnschichttechnik hergestelltes, insbesondere gesputtertes, Strukturelement. Besonders bevorzugt ist es, wenn die Wandung, insbesondere die perforierte Struktur, eine geflochtene Gitterstruktur aufweist. Dabei kann die geflochtene Gitterstruktur wenigstens 8, insbesondere wenigstens 12, insbesondere wenigstens 16, insbesondere wenigstens 18, insbesondere wenigstens 20, insbesondere wenigstens 24, insbesondere wenigstens 32, insbesondere wenigstens 36, insbesondere wenigstens 38, fadenförmige Elemente umfassen. Die vorgenannten Ausführungsbeispiele haben den gemeinsamen Vorteil einer einfachen Herstellung, wobei die Verwendung einer geflochtenen Gitterstruktur zusätzlich eine besonders geeignete Feinmaschigkeit ermöglicht. Dadurch wird eine besonders effektive Rekanalisationswirkung erreicht, wobei gleichzeitig durch die feinen Maschen sichergestellt ist, dass sich möglicherweise ablösende Thrombusbestandteile nicht in den Strömungskanal bzw. die Strömungspassage begeben. Ein weiterer Vorteil der geflochtenen Gitterstruktur besteht darin, dass damit eine relativ hohe Flexibilität des Hohlkörpers erreicht wird. Dadurch ist es besonders effizient möglich, das Rekanalisationselement in einen komprimierten, insbesondere gestreckten, und einen expandierten, insbesondere spiralförmigen, Zustand zu überführen.

Erfindungsgemäß ist vorgesehen, dass das Rekanalisationselement, insbesondere der Hohlkörper, von einem komprimierten Zustand in einen expandierten Zustand überführbar ist. Vorteilhafterweise weist der Hohlkörper im komprimierten Zustand innerhalb eines Zufuhrsystems ein geradliniges bzw. zylinderförmiges Profil auf. Bei der Expansion nimmt der Hohlkörper bzw. das Rekanalisationselement die spiralförmige Windung an. Dabei kann die spiralförmige Windung unterschiedliche Steigungen aufweisen. Eine größere Steigung hat den Vorteil, dass die Länge des Hohlkörpers, also die Längserstreckung des Hohlkörpers im komprimierten Zustand, vergleichsweise klein ist. Das bedeutet, dass auch das Verhältnis zwischen Länge des Rekanalisationselements, also Länge über alle Windungen, zur Länge des Hohlkörpers verhältnismäßig klein ist, wobei der Hohlkörper grundsätzlich eine größere Länge aufweist als das gesamte Rekanalisationselement.

Der Vorteil eines relativ kleinen Längenverhältnisses bzw. eines relativ kurzen Hohlkörpers besteht darin, dass die Reibung zwischen der Wandung des Hohlkörpers im komprimierten Zustand und einem Zufuhrsystem reduziert ist, da die Berührungsfläche zwischen Zufuhrsystem und Hohlkörper relativ klein ist. Die Schiebekraft bzw. Zugkraft, die erforderlich ist, um das Rekanalisationselement bzw. den Hohlkörper im Zufuhrsystem zu bewegen, ist daher relativ gering, wodurch die Handhabbarkeit verbessert wird. Um dies zu erreichen, hat es sich als besonders vorteilhaft erwiesen, wenn die spiralförmige Windung eine Steigung von wenigstens 10 mm, insbesondere wenigstens 15mm, insbesondere wenigstens 20 mm, insbesondere wenigstens 25 mm, insbesondere wenigstens 30 mm, aufweist. Eine Steigung von wenigstens 30 mm ist besonders vorteilhaft bei einem Einsatz der medizinischen Vorrichtung in Körpergefäßen mit einem Gefäßdurchmesser von höchstens 3 mm. Bei Gefäßdurchmessern von höchstens 5 mm, insbesondere im Bereich zwischen 3 mm und 5 mm, hat sich eine Steigung der spiralförmigen Windung von wenigstens 30 mm, insbesondere wenigstens 40 mm, insbesondere wenigstens 50 mm als vorteilhaft erwiesen. Bei Gefäßen bzw. Hohlräumen, die einen Querschnittsdurchmesser aufweisen, der größer als 5 mm beträgt, wird vorzugsweise eine medizinische Vorrichtung eingesetzt, bei der die spiralförmige Windung des Hohlkörpers eine Steigung von wenigstens 30 mm, insbesondere wenigstens 40 mm, insbesondere wenigstens 50 mm, insbesondere wenigstens 60 mm, insbesondere wenigstens 70 mm, insbesondere wenigstens 80 mm, insbesondere wenigstens 90 mm, insbesondere wenigstens 100 mm aufweist.

Alternativ kann es vorteilhaft sein, wenn das Rekanalisationselement eine höhere Anhaftaffinität zum Thrombus bzw. allgemein zu dem Konkrement aufweist. Dies kann beispielsweise durch eine höhere Anzahl von spiralförmigen Windungen erreicht werden. Dabei weisen die einzelnen Windungen eine vergleichsweise kleine Steigung auf. Besonders bevorzugt ist eine Steigung der spiralförmigen Windung von höchstens 30 mm, insbesondere höchstens 20 mm, insbesondere höchstens 10 mm, insbesondere höchstens 8 mm, insbesondere höchstens 7 mm, insbesondere höchstens 6 mm, insbesondere höchstens 5 mm, insbesondere höchstens 3 mm, insbesondere höchstens 2 mm. Dabei beziehen sich Angaben hinsichtlich der Steigung der spiralförmigen Windung im Rahmen dieser Anmeldung generell auf eine volle Windung, also eine Windung, die einen Umlaufwinkel von 360° aufweist. Die Angabe der Steigung bezieht sich also generell auf eine volle Umdrehung des Hohlkörpers um eine zentrale Windungsachse.

Um die vorgenannten Vorteile und Wirkungen zu erreichen, ist es ferner bevorzugt, wenn das Verhältnis zwischen der Steigung der spiralförmigen Windung und einem Querschnittsdurchmesser des Hohlkörpers höchstens 1000 %, insbesondere höchstens 200 %, insbesondere höchstens 180 %; insbesondere höchstens 160 %, insbesondere höchstens 150 %, insbesondere höchstens 140 %, insbesondere höchstens 130 %, insbesondere höchstens 120 %, beträgt. Die spiralförmige Windung kann außerdem einen Steigungswinkel aufweisen, der wenigstens 45°, insbesondere wenigstens 60°, insbesondere wenigstens 70°, insbesondere wenigstens 80°, beträgt. Alternativ kann der Steigungswinkel höchstens 45°, insbesondere höchstens 40°, insbesondere höchstens 35°, insbesondere höchstens 30°, insbesondere höchstens 25°, insbesondere höchstens 20°, betragen.

Bei einer bevorzugten Ausführungsform der erfindungsgemäßen Vorrichtung weist der Hohlkörper im expandierten Zustand zumindest abschnittsweise unterschiedliche Querschnittsdurchmesser auf. Das ermöglicht einen besonders flexiblen Einsatz des Rekanalisationselements.

Vorzugsweise weist der Hohlkörper im expandierten Zustand an einem distalen Ende einen größeren Querschnittsdurchmesser als in einem proximalen Abschnitt auf. Eine derartige Konfiguration ermöglicht eine besonders stabile Anhaftung des Hohlkörpers bzw. allgemein des Rekanalisationselements an einem Konkrement. Überdies ermöglicht der größere Querschnittsdurchmesser an einem distalen Ende eine verbesserte Abdichtung des Rekanalisationselements mit einer Gefäßwand, insbesondere beim Zurückziehen des Rekanalisationselements, wobei das Konkrement durch größer werdende Körperhohlräume gezogen wird. Gleichzeitig wird die Reibung des Rekanalisationselements an der Gefäßwand reduziert. Eine Verletzung von Gefäßen, insbesondere ein Reizung der Gefäßwände, wird somit vermieden.

Eine besonders bevorzugte Ausgestaltung der erfindungsgemäßen Vorrichtung sieht vor, dass der Hohlkörper im expandierten Zustand an einem distalen Ende des Rekanalisationselements einen Filterabschnitt bildet. Der Filterabschnitt kann beispielsweise durch einen vergrößerten Querschnittsdurchmesser am distalen Ende gebildet sein. Der Hohlkörper bzw. die Wandung des Hohlkörpers kann also an einem distalen Ende eine Filterfunktion übernehmen, so dass sich ablösende Konkrementpartikel aufgefangen werden. Die Bildung von Emboli bzw. weiteren Gefäßverschlüssen, insbesondere stromabwärts des Behandlungsortes, wird dadurch vermieden.

Vorzugsweise ist das Rekanalisationselement proximal mit einem Zufuhrelement, insbesondere einem Führungsdraht, verbunden. Das Zufuhrelement ermöglicht auf einfache Weise die sichere Handhabung des Rekanalisationselements. Überdies ermöglicht das Zufuhrelement, insbesondere der Führungsdraht, eine haptische Rückmeldung an den Anwender des Rekanalisationselements, so dass eine feinfühlige und genaue Rekanalisation ermöglicht ist.

Das Rekanalisationselement kann ferner eine einen Wirkstoff freisetzende, insbesondere antithrombogene, Beschichtung aufweisen. Dadurch wird auf besonders vorteilhafte Weise die mechanische Rekanalisation und Entfernung eines Konkrements mit einer medikamentösen Behandlung unterstützt. Bei der Entfernung von Thromben aus Blutgefäßen kann insbesondere eine antithrombogene Beschichtung vorteilhaft sein, die eine Blutgerinnung im Bereich der porösen Struktur der Wandung des Hohlkörpers bzw. in den Maschen der Gitterstruktur verhindert und die Aufrechterhaltung der rekanalisierten Strömungspassage sicherstellt.

Bei einer weiteren bevorzugten Ausführungsform ist vorgesehen, dass das Rekanalisationselement zumindest abschnittsweise eine fluiddichte Abdeckung aufweist. Die Abdeckung kann beispielsweise einen Kunststoff, vorzugsweise Polyurethan oder Elasteon, umfassen. Die Abdeckung kann sich sowohl abschnittsweise als auch vollständig über das Rekanalisationselement, insbesondere den Hohlkörper bzw. die Wandung des Hohlkörpers, erstrecken. Durch die fluiddichte Abdeckung wird das Risiko von weiteren Gefäßverschlüssen durch Ablösen von Konkrementpartikeln vermindert.

Die fluiddichte Abdeckung kann ein oder mehrere Saugöffnungen umfassen, die im Gebrauch einem Konkrement zugewandt sind. Die Saugöffnungen sind also vorteilhafterweise auf einem Innenumfang bzw. einer Innenkontur des Rekanalisationselements angeordnet. Beispielsweise kann die Wandung des Hohlkörpers eine fluiddichte Beschichtung umfassen, wobei in einer den Innenumfang des gesamten Rekanalisationselements definierten Wandungsebene die Saugöffnungen angeordnet sind. Die Saugöffnungen können beispielsweise durch Wandungsbereiche gebildet sein, die keine Abdeckung aufweisen bzw. in denen die Abdeckung geöffnet ist, so dass die Wandungsstruktur, beispielsweise eine geflochtene Gitterstruktur, die als Trägerstruktur für die Abdeckung dient, freigelegt ist. Generell ist vorgesehen, dass die Saugöffnungen eine Fluidverbindung zwischen dem Hohlraum, der durch den Hohlkörper gebildet ist, und dem Innenraum des Rekanalisationselements, also dem durch die spiralförmig gewundene Struktur begrenzten Raumvolumen, in dem das Konkrement im Gebrauch angeordnet ist, bildet. Durch die Saugöffnungen wird erreicht, dass das Konkrement an das Rekanalisationselement angesaugt bzw. umgekehrt das Rekanalisationselement, insbesondere der Hohlkörper, an das Konkrement angesaugt wird. Damit wird sichergestellt, dass sich das Konkrement nicht vom dem Rekanalisationselement löst, insbesondere beim Abtransport des Konkrements durch größere Körperhohlräume.

Bei einer weiteren bevorzugten Ausführungsform ist das Rekanalisationselement an einem distalen Ende mit einem Betätigungselement verbunden. Das Betätigungselement erstreckt sich im Wesentlichen längsaxial entlang des Rekanalisationselements. Vorteilhafterweise verläuft das Betätigungselement zumindest teilweise entlang einer Außenkontur und/oder entlang einer Innenkontur des Rekanalisationselements. Mit Hilfe des Betätigungselements wird ermöglicht, dass das Rekanalisationselement in längsaxialer Richtung im Sinne einer Längenänderung betätigbar ist. Durch das Betätigungselement kann das Rekanalisationselement also in Längsrichtung komprimiert werden bzw. allgemein die Steigung der spiralförmigen Windung beeinflusst werden. Beispielsweise kann das Rekanalisationselement durch das Betätigungselement derart betätigbar sein, dass das Rekanalisationselement im Gebrauch streckbar ist. Auf diese Weise ändert sich der Innendurchmesser, also die Dimension der Innenkontur, des Rekanalisationselements, wodurch eine verbesserte Anhaftung bzw. Fixierung des Konkrements erreichbar ist. Dabei kann das Betätigungselement längsaxial durch das Rekanalisationselement, also entlang einer Innenkontur, oder außerhalb des Rekanalisationselements, insbesondere entlang einer Außenkontur, verlaufen.

Gemäß einem nebengeordneten Aspekt beruht die Erfindung auf dem Gedanken, ein Set anzugeben, das die zuvor beschriebene medizinische Vorrichtung und ein Zufuhrsystem für eine derartige Vorrichtung umfasst. Aus Sterilitätsgründen ist ein derartiges Set vorteilhaft.

Die Erfindung wird im Folgenden anhand von Ausführungsbeispielen unter Bezugnahme auf die beigefügten, schematischen Zeichnungen näher erläutert. Darin zeigen:
- Fig. 1:: eine Seitenansicht einer erfindungsgemäßen medizinischen Vorrichtung gemäß einem bevorzugten Ausführungsbeispiel im Gebrauch;
- Fig. 2:: eine Seitenansicht einer erfindungsgemäßen medizinischen Vorrichtung gemäß einem weiteren Ausführungsbeispiel im Gebrauch;
- Fig. 3a und 3b:: jeweils eine Seitenansicht der medizinischen Vorrichtung gemäß Fig. 2 mit unterschiedlichen Steigungen der spiralförmigen Windung; und
- Fig. 4 bis 7:: jeweils eine Seitenansicht einer erfindungsgemäßen medizinischen Vorrichtung gemäß einem weiteren Ausführungsbeispiel im Gebrauch;
- Fig. 8: eine Tabelle mit bevorzugten Größenverhältnissen der medizinischen Vorrichtung und
- Fig. 9: eine Seitenansicht einer medizinischen Vorrichtung nach einem weiteren erfindungsgemäßen Ausführungsbeispiel mit verschlossenem distalen Ende.

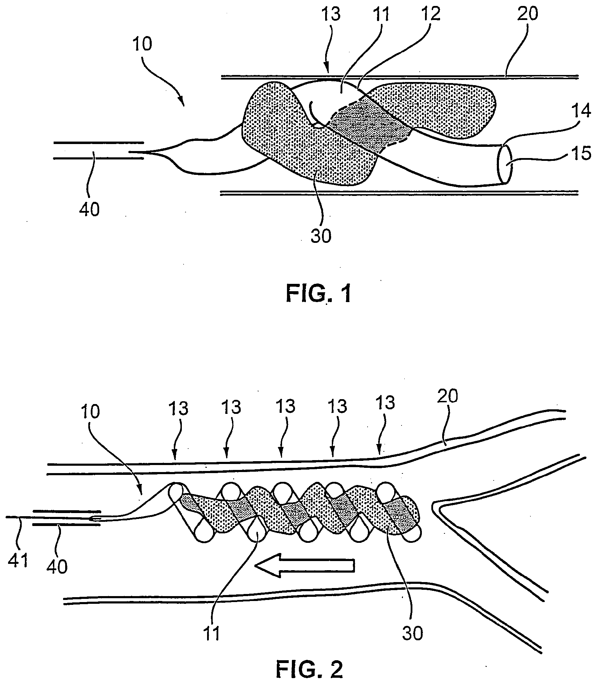

In Fig. 1 ist ein Ausführungsbeispiel der medizinischen Vorrichtung gezeigt, wobei die medizinische Vorrichtung ein Rekanalisationselement 10 aufweist, das im Gebrauch, wie in Fig. 1 dargestellt, einen expandierten Zustand aufweist. Dabei bildet das Rekanalisationselement 10 im expandierten Zustand einen Hohlkörper 11, der im Wesentlichen rohrförmig ausgebildet ist. Das Rekanalisationselement 10 bildet mit anderen Worten einen Hohlkanal, der durch eine rohrförmige Wandung 12 begrenzt ist. Der Hohlkörper 11 bzw. allgemein das Rekanalisationselement 10 ist ferner spiralförmig gewunden, wobei das Rekanalisationselement 10 bzw. der Hohlkörper 11 bei dem Ausführungsbeispiel gemäß Fig. 1 eine einzige spiralförmige Windung 13 aufweist. Der Hohlkörper 11 bzw. allgemein das Rekanalisationselement 10 bildet eine Spirale.

Die medizinische Vorrichtung ist in Fig. 1 im Gebrauch dargestellt, also im Zusammenwirken mit einem Konkrement 30 in einem Körperhohlraum 20. Der Körperhohlraum 20 kann beispielsweise ein Blutgefäß umfassen. In diesem Fall bildet das Konkrement 30 vorzugsweise ein Blutgerinnsel bzw. einen Thrombus. Durch die spiralförmige Windung 13 wird das Rekanalisationselement 10 mit dem Konkrement 30 verschränkt bzw. allgemein formschlüssig mit dem Konkrement 30 verbunden. Vorzugsweise umhüllt das Rekanalisationselement 10 bzw. der Hohlkörper 11 das Konkrement 30.

Um eine effiziente Rekanalisation zu bewirken, weist das Rekanalisationselement 10 bzw. der Hohlkörper 11 an einem distalen Ende 14 eine axiale Öffnung 15 auf. Ferner kann das Rekanalisationselement 10 an einem proximalen Ende bzw. in einem proximalen Abschnitt 18 eine Öffnung aufweisen, so dass Fluide vollständig durch den Hohlkörper 11 strömen können. Die proximale Öffnung kann im Wesentlichen radial bezüglich des Hohlkörpers 11 ausgerichtet sein. Vorzugsweise ist die proximale Öffnung in der Wandung 12 angeordnet. Es ist auch möglich, dass die Wandung 12 des Hohlkörpers 11 eine perforierte Struktur aufweist. Das bedeutet, dass die Wandung 12 zumindest abschnittsweise ein oder mehrere Öffnungen umfasst, durch die Körperfluide, insbesondere Blut in den Hohlkörper 11 einströmen bzw. allgemein durch die Wandung 12 hindurchströmen können. Zumindest im proximalen Abschnitt 18 des Rekanalisationselements kann eine derartige perforierte Struktur vorgesehen sein.

Generell kann der Hohlkörper 11 wenigstens eine Eintrittsöffnung und wenigstens eine Austrittsöffnung umfassen, um die Durchströmbarkeit des Hohlkörpers 11 sicherzustellen. Die Eintritts- und/oder Austrittsöffnungen können sowohl axiale Öffnungen, als auch radiale Öffnungen, die in der Wandung 12 ausgebildet sind, umfassen. Die Durchströmbarkeit des Hohlkörpers 11 kann auf verschiedene Arten erfolgen. Wenn der Hohlkörper in der Form eines Gittergeflechts ohne Abdeckung ausgebildet ist, wird durch die Spiralform des Hohlkörpers ein Ringkanal gebildet, der sich um das mittig vom Hohlkörper 11 gehaltene Konkrement erstreckt. Die Eintritts- und Austrittsöffnung des Höhlkörpers 11 erfolgt somit durch die Zellöffnungen bzw. Maschen der Gitterstruktur, die die Wandung des Hohlkörpers 11 bildet. Der Hohlkörper 11 ist somit seitlich durchströmbar. Wie beispielsweise in den Figuren 1 bis 6 gezeigt, weist das proximale Ende 21 des Hohlkörpers 11 einen Übergangsbereich 21a auf, der die von der Katheterspitze in distaler Richtung gesehene erste Windung 13 des spiralförmigen Hohlkörpers 11 mit dem Führungsdraht 41 verbindet. Der Übergangsbereich 21a verjüngt sich in proximaler Richtung derart, dass die Drähte bzw. allgemein die die Gitterstruktur bildenden Gitterelemente zusammenlaufen und in den Führungsdraht übergehen oder mit diesem verbunden sind. In distaler Richtung weitet sich der Übergangsbereich 21a bis der Durchmesser des Übergangsbereichs 21a dem Durchmesser der ersten Windung 13 der Spirale des Hohlkörpers 11 entspricht. Im Übergangsbereich 21a ist die Wandung zipfelartig ausgebildet.

Der Übergangsbereich 21a kann sich axial erstrecken. Dies bedeutet, dass der Übergangsbereich 21a eine im Wesentlichen geradlinig verlaufende und daher nicht spiralförmig gekrümmte Mittelachse aufweist. Diese Form ist nicht zwingend. Der Übergangsbereich 21a kann eine Spirale bilden, die die Spiralform des Hohlkörpers 11 im Bereich der Windungen 13 fortsetzt. Im Übergangsbereich 21a nimmt der Außendurchmesser der Spirale in proximaler Richtung ab. Dabei kann die Spiralform des Übergangsbereichs 21a am distalen Ende des Führungsdrahtes 41 beginnen.

Aufgrund der sich verjüngenden Form des Übergangsbereichs 21a wird im implantierten Zustand die Wandung seitlich angeströmt. Die Blutströmung gelangt durch die Zellöffnungen der Gitterstruktur ins Lumen des Hohlkörpers 11. In diesem Sinne ist der Hohlkörper 11 durchströmbar. Die Eintrittsöffnungen bilden dabei keine geometrisch definierte einheitliche Gesamtöffnung. Die Gesamtöffnung für den Eintritt der Blutströmung setzt sich aus den seitlich angeströmten bzw. seitlich anströmbaren Zellöffnungen der Gitterstruktur im Übergangsbereich 21a zusammen und ist in diesem Sinne geometrisch undefiniert.

Durch den Übergangsbereich 21a wird überdies erreicht, dass zwischen dem Zufuhrsystem 40 bzw. dem Führungsdraht 41 einerseits und der ersten Windung 13 andererseits ein Abstand gebildet ist. Dieser Abstand bewirkt, dass im implantierten Zustand ein freier, d.h. vom Konkrement nicht überdeckter Abschnitt des Hohlkörpers 11 vorhanden ist, der als Eintrittsöffnung bzw. als eine Vielzahl von Eintrittsöffnungen (Zellöffnungen) fungiert.

Im Übergangsbereich 21a können im Fall eines Geflechts die Drähte paarweise oder mehrfach zusammengeführt sein, um die Zellöffnungen zu vergrößern. Die zusammengeführten Drähte können verdrillt sein.

Zusätzlich oder alternativ kann das proximale Ende des Hohlkörpers eine geometrisch definierte Eintrittsöffnung aufweisen, die einen quer zur Blutströmung bzw. zur Mittelachse des Hohlkörpers verlaufenden Durchströmungsquerschnitt bildet. Die Eintrittsöffnung kann sich geneigt zur Mittelachse des Hohlkörpers 11 erstrecken und einen wiedereinziehbare Abschlusskante bilden. Ein Beispiel hierfür ist in den Figuren 2 bis 13 und den zugehörigen Beschreibungstextstellen, insbesondere auf S. 15 bis 31 der DE 10 2009 056 450 offenbart, die auf die Anmelderin zurückgeht und die durch Verweis in diese Anmeldung mit eingeschlossen ist.

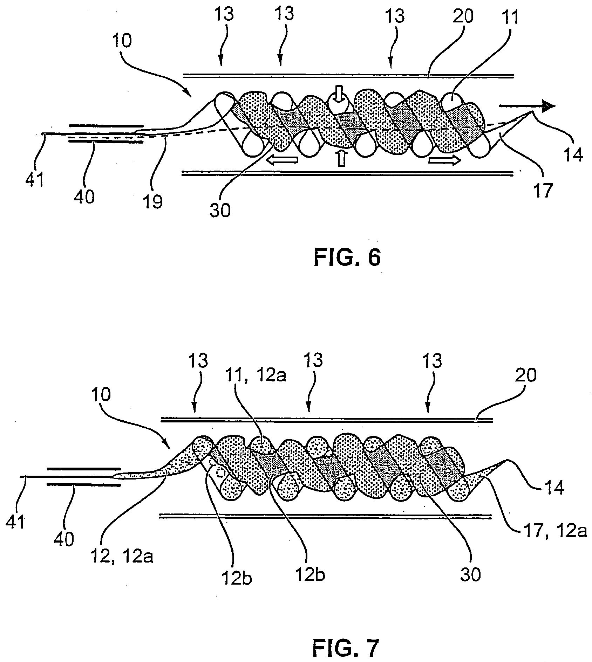

Das distale Ende 22 kann in entsprechender Weise wie das proximale Ende 21 ausgeführt sein, s. Fig. 6, wobei sich die Verjüngung in distaler Richtung erstreckt. Die vorgenannten Merkmale des Übergangsbereichs 21a werden auch im Zusammenhang mit dem distalen Ende 22 offenbart, wobei die Spitze des distalen Bereichs 22 frei ist.

Anstelle der Verjüngung gemäß Fig. 6 kann das distale Ende 22 eine Aufweitung aufweisen, deren Durchmesser größer ist als der Durchmesser des Hohlkörpers 11 im mittleren Bereich des Rekanalisationselements 10. Es ist auch möglich, dass der Durchmesser des Hohlkörpers 11 entlang der Spirale konstant bleibt. Diese Formen des distalen Endes 22 werden im Zusammenhang mit einer Gitterstruktur ohne Abdeckung offenbart, deren Wandung seitlich durchströmbar ist sowie im Zusammenhang mit einer Gitterstruktur mit Abdeckung.

Die Eintrittsöffnung bzw. die Austrittsöffnung kann am proximalen bzw. distalen Ende des Hohlkörpers 11 im Sinne einer Rohröffnung vorgesehen sein. Die Öffnung ist geometrisch definiert und weist beispielsweise ein kreisförmiges Querschnittsprofil auf. Dabei kann vorgesehen sein, dass die Eintrittsöffnung und/oder die Austrittsöffnung verschließbar sind. Die Verschließbarkeit beispielsweise der Austrittsöffnung hat den Vorteil, dass Medikamente in den Hohlkörper eingebracht werden können, die dort während einer bestimmten Zeitdauer verbleiben und dort auf den Thrombus bzw. das eingefangene Konkrement einwirken. Danach kann die Austrittsöffnung geöffnet werden, so dass das Gefäß rekanalisiert wird. Die vorstehende Beschreibung der Eintritts- bzw. Austrittsöffnung wird im Zusammenhang mit allen Ausführungsbeispielen bzw. generell im Zusammenhang mit der Erfindung offenbart.

Das Körperfluid kann durch die perforierte Struktur im proximalen Abschnitt 18 in den Hohlkörper einströmen und das Konkrement 30 durch den Hohlkanal, der durch den Hohlkörper 11 gebildet ist, passieren. An der axialen Öffnung 15 kann das Körperfluid in einem Bereich austreten, der stromabwärts des Konkrements 30 angeordnet ist. Auf diese Weise ist das Körpergefäß bzw. der Körperhohlraum 20 rekanalisiert. Die perforierte Struktur kann sich auch über weitere Abschnitte des Hohlkörpers 11, beispielsweise über die gesamte Wandung 12, erstrecken. Insbesondere ist es vorteilhaft, wenn die Wandung 12 vollständig eine geflochtene Gitterstruktur aufweist. Eine geflochtene Gitterstruktur ermöglicht eine besonders kleine Komprimierung des Rekanalisationselements 10 und erleichtert dessen Zufuhr in einen kleinen Körperhohlraum. Ferner kann durch die geflochtene Gitterstruktur eine besonders hohe Feinmaschigkeit erreicht werden, so dass ein Eindringen von Konkrementpartikeln in den Hohlkörper 11 und somit in den rekanalisierten Strömungskanal vermieden wird.

Hinsichtlich der Feinmaschigkeit ist es vorteilhaft, wenn die Maschengröße bzw. Porengröße höchstens 1 mm, insbesondere höchstens 0,8 mm, insbesondere höchstens 0,6 mm, insbesondere höchstens 0,5 mm, insbesondere höchstens 0,4 mm, insbesondere höchstens 0,3 mm, insbesondere höchstens 0,2 mm, beträgt. Dabei entspricht die Porengröße dem Durchmesser eines virtuellen, größtmöglichen Kreises, der in der Umfangsebene bzw. Wandungsebene des Hohlkörpers 11 in eine Masche bzw. Zelle hineinbeschrieben werden kann. Die Porengröße bzw. Maschengröße kann dabei im distalen Bereich des Hohlkörpers 11, insbesondere in Filterabschnitt 16 größer oder kleiner sein als in einem mittleren Abschnitt bzw. funktionalen Bereich des Rekanalisationselements 10. Die hohe Feinmaschigkeit, also die vorgenannten, kleinen Porengrößen, ermöglichen eine Verbesserung der Filterfunktion. Überdies wird mit einer derartigen kleinen Porengröße bzw. Maschengröße die Durchlässigkeit der Wandung 12 für Körperfluide, insbesondere Blut, reduziert, so dass die Rekanalisationsströmung vorzugsweise entlang der Wandung 12, insbesondere innerhalb des durch den Hohlkörper 11 gebildeten Hohlkanals, geleitet wird.

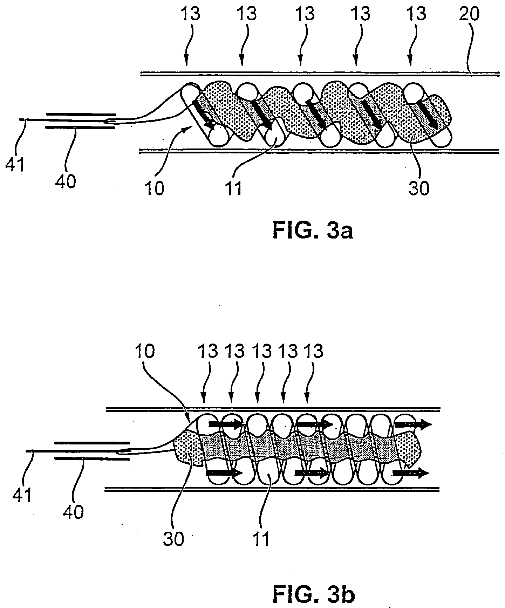

Es ist auch möglich, dass die Porengröße bzw. Maschengröße wenigstens 0,2 mm, insbesondere wenigstens 0,4 mm, insbesondere wenigstens 0,6 mm, insbesondere wenigstens 0,8 mm, insbesondere wenigstens 1,0 mm, insbesondere wenigstens 1,2 mm, insbesondere wenigstens 1,5 mm, insbesondere wenigstens 2,0 mm, beträgt. Dadurch wird ein höherer Durchfluss durch die Wandung 12 bzw. die Maschen der Gitterstruktur, die die Wandung 12 bilden, ermöglicht. Auf diese Weise kann eine zusätzliche Strömungspassage für die Rekanalisationsströmung bereitgestellt werden, die sich beispielsweise zwischen dem Konkrement 30 und der Gefäßwand des Körperhohlraums 20 erstreckt (Fig. 3b).

In diesem Zusammenhang wird darauf hingewiesen, dass das Rekanalisationselement 10 im komprimierten Zustand bzw. im gecrimpten Zustand vorzugsweise eine im Wesentlichen geradlinige Struktur aufweist. Die spiralförmige Windung 13 bildet sich erst bei der Expansion des Rekanalisationselements 10. Es ist aber auch möglich, dass das Rekanalisationselement 10 im komprimierten Zustand eine Spiralform aufweist, die beispielsweise gegenüber der Spiralform im dem expandierten Zustand weniger ausgeprägt ist.

Im komprimierten bzw. gecrimpten Zustand des Rekanalisationselements 10 bildet der Hohlkörper 11 vorteilhafterweise eine drahtbündelähnliche Querschnittsstruktur. Das bedeutet, dass der Hohlkörper 11 im Wesentlichen keinen durchströmbaren Hohlkanal bildet. Der Hohlkanal entsteht vielmehr durch die Aufweitung des Hohlkörpers 11, insbesondere der Gitterstruktur des Hohlkörpers 11, während der Expansion des Rekanalisationselements 10. Der Hohlkörper 11 verhält sich also stentähnlich.

Insgesamt kann das Rekanalisationselement 10 eine Rückstellkraft bzw. Radialkraft aufweisen, die die Expansion bewirkt oder zumindest unterstützt. Die Rückstellkraft wirkt sowohl auf den Hohlkörper 11, als auch auf die spiralförmige Windung 13. Dabei wird Rückstellkraft bzw. Radialkraft bei der Expansion wirksam und ermöglicht bzw. unterstützt die Bildung des Hohlkanals im Hohlkörper 11 und die Bildung der spiralförmigen Windung 13 des Rekanalisationselements 10. Mit anderen Worten wird durch die Expansion der Rekanalisationsvorrichtung sowohl der Gesamtdurchmesser der Vorrichtung, d.h. der Außendurchmesser des spiralförmig gewundenen Hohlkörpers 11 als auch der Querschnittsdurchmessers des Hohlkörpers 11 selbst vergrößert.

Im Allgemeinen kann das Rekanalisationselement 10 unterschiedliche Konfigurationen umfassen. Beispielsweise kann das Rekanalisationselement 10 in einem - in Bezug auf die Axialrichtung - mittleren Abschnitt eine geflochtene Gitterstruktur umfassen. Alternativ oder zusätzlich kann die geflochtene Gitterstruktur auch in einem distalen Abschnitt 17 des Rekanalisationselement 10 vorgesehen sein. Im proximalen Abschnitt 18 kann die perforierte Struktur größere Öffnungen, insbesondere eine größere Maschenweite als im mittleren Abschnitt des Rekanalisationselements 10 aufweisen. Die Öffnungen im mittleren Abschnitt sind vorteilhafterweise derart angepasst, dass einerseits ein Medikament direkt an das Konkrement 30 gelangen kann und andererseits eine Filterfunktion erreicht wird, so dass Konkrementpartikel einer bestimmten Größe und - insbesondere beim Einsatz der medizinischen Vorrichtung in Blutgefäßen - Blutpartikel einer bestimmten Größe effizient zurückgehalten werden. Hingegen können die Öffnungen der perforierten Struktur im proximalen Abschnitt 18 vergleichsweise größer ausgebildet sein, um einen möglichst widerstandsarmen Durchfluss zu gewährleisten.

Um die Größe der Öffnungen bzw. die Maschengröße in den unterschiedlichen Abschnitten des Rekanalisationselements 10 zu erreichen, ist es vorteilhaft, wenn die im mittleren Abschnitt die geflochtene Gitterstruktur bildenden fadenförmigen Elemente im proximalen Abschnitt 18 eine andere Flechtart aufweisen oder ungeflochten sind. Beispielsweise kann die geflochtene Gitterstruktur im mittleren Abschnitt eine Flechtart aufweisen, die als 1-über-1 oder 1-über-2 oder 1-über-3 bezeichnet wird. Bei einer festgelegten Anzahl von fadenförmigen Elementen bzw. Drähten wird dadurch eine relativ kleine Rautengröße der Gittergeflechtmaschen erreicht. Im proximalen Abschnitt 18 kann hingegen eine Flechtart gewählt sein, die im Wesentlichen einer 2-über-2 oder 3-über-3 oder 4-über-4 oder 5-über-5-Flechtung entspricht. Dabei können sich die Drähte bzw. allgemein fadenförmigen Elemente paarweise oder in Dreier- bzw. Vierergruppen nebeneinander anordnen, wodurch die Maschengröße bzw. Rautengröße erhöht wird. Die erhöhte Maschengröße kann einen verbesserten Durchfluss, insbesondere Blutdurchfluss, bewirken.

Hinsichtlich der Flechtarten ist es besonders bevorzugt, wenn zwei oder mehr fadenförmige Elemente, insbesondere Drähte, die die Wandung 12 bilden, im proximalen Abschnitt 18 zusammengeführt sind. Die zusammengeführten Drähte können im proximalen Abschnitt 18 verdrillt, miteinander verflochten oder andersartig miteinander verbunden sein. Dabei ist es unerheblich, ob die zusammengeführten Drähte im mittleren Abschnitt des Hohlkörpers 11 gegenläufig oder gleichläufig angeordnet sind. Im proximalen Abschnitt 18 verlaufen die zusammengeführten Drähte bzw. fadenförmigen Elemente in Form einer Drahtgruppe vorzugsweise in axialer Richtung bzw. parallel zur Mittelachse des Hohlkörpers 11. Die Mittelachse des Hohlkörpers 11 ist im expandierten Zustand spiralförmig gewunden bzw. folgt der spiralförmigen Windung 13. Es ist auch möglich, dass die Drahtgruppe in einem Winkel zur Mittelachse des Hohlkörpers 11 verläuft. Das bedeutet, dass die Drahtgruppe in der Wandungsebene eine Spiralform beschreibt. Vorzugsweise sind im proximalen Abschnitt 18 mehrere Drahtgruppen vorgesehen, die in die gleiche Richtung verlaufen. Es ist nicht ausgeschlossen, dass die Drahtgruppen in der Wandung 12 in unterschiedlichen Richtungen angeordnet sind. Die Drahtgruppen können sich auch überkreuzen. Vorzugsweise verlaufen die Drahtgruppen im proximalen Abschnitt 18 allerdings derart, dass eine Überkreuzung vermieden ist. Dazu ist eine parallele bzw. annähernd parallele Anordnung der Drahtgruppen zweckmäßig.

Der proximale Abschnitt 18 kann trichterförmig ausgebildet sein. Die Drahtgruppen bzw. die Gruppen der fadenförmigen Elemente können im proximalen Abschnitt 18 bzw. zu einem proximalen Ende des Hohlkörpers 11 hin in Richtung der Mittelachse des Hohlkörpers 11 zusammenlaufen. Im Bereich der Mittelachse können die die Wandung 12 bildenden fadenförmigen Elemente bzw. die den proximalen Abschnitt 18 bildenden Drahtgruppen zu einem gemeinsamen Strang verbunden sein. Der so gebildete gemeinsame Strang kann ferner mit einem Zufuhrelement, insbesondere einem Führungsdraht 41, fest verbunden oder verbindbar sein. Es ist auch möglich, dass der proximale Strang mit dem Zufuhrelement bzw. dem Führungsdraht 41 einteilig ausgebildet ist.

In einer besonders bevorzugten Konfiguration sind im proximalen Abschnitt 18 jeweils zwei Drähte, die im mittleren Abschnitt in unterschiedlichen Windungsrichtungen angeordnet sind, zu einer Drahtgruppe verdrillt. Im Bereich der proximalen Trichterspitze, d.h. am proximalen Ende des Hohlkörpers 11, kann die Verdrillung der zwei Drähte aufgelöst sein. Die beiden Drähte verlaufen dann beide in den proximal gebildeten Strang, allerdings nicht in der ursprünglichen verdrillten Anordnung. Vielmehr kann einer der beiden im proximalen Abschnitt 18 verdrillten Drähte im proximalen Strang in einer ersten Richtung verlaufen. Der zweite Draht ist im proximalen Strang in einer anderen, insbesondere gegenläufigen, Richtung angeordnet. Beispielsweise können alle Drähte bzw. fadenförmigen Elemente, die im Bereich des proximalen Endes des Hohlkörpers 11 auf der Mittelachse zusammengeführt sind und ursprünglich in derselben Richtung verlaufen, zu einem inneren proximalen Strang verdrillt sein. Die anderen Drahtelemente bzw. fadenförmigen Elemente, die eine gegenläufige Anordnung aufweisen, können um den inneren Strang herumgewickelt bzw. verdrillt sein und einen, den inneren Strang umhüllenden, äußeren Strang bilden, wobei die Wickelrichtung bzw. Verdrillrichtung im äußeren Strang vorzugsweise gegenüber dem inneren Strang umgekehrt ist. Der proximale Strang ist also im Querschnitt zweischichtig aufgebaut, wobei sich die beiden Schichten durch die unterschiedliche Verdrillrichtung unterscheiden. Auf diese Weise ergibt sich eine besonders stabile Konfiguration des proximalen Strangs, wodurch eine gute Verbindung des verformbaren bzw. expandierbaren Hohlkörpers mit dem Zufuhrelement, insbesondere dem Führungsdraht 41, erreicht wird.

Die Positionierung des Rekanalisationselements 10 in einem Körperhohlraum 20 erfolgt vorzugsweise durch Zufuhr des Rekanalisationselements 10 über ein Zufuhrsystem 40, wie in Fig. 1 dargestellt. Das Zufuhrsystem 40 kann einen medizinischen Katheter umfassen. Während der Behandlung kann das Rekanalisationselement 10 mit dem Zufuhrsystem 40, insbesondere dem Führungsdraht 41, verbunden sein bzw. verbunden bleiben, so dass durch Zurückziehen des Zufuhrsystems 40 das mit dem Rekanalisationselement 10 gekoppelte Konkrement 30 aus dem Körperhohlraum 20 entfernbar ist. Es ist ferner möglich, das Rekanalisationselement 10 durch entsprechende Bewegung des Zufuhrsystems 40 bzw. des Führungsdrahts 41 in das Zufuhrsystem 40 zurückzuziehen und somit zu repositionieren. Durch gleichzeitiges Ziehen des Führungsdrahts 41 und des Zufuhrsystems 40 kann das Konkrement 30, insbesondere ein Thrombus, entfernt werden, ohne das Konkrement 30 in das Zufuhrsystem 40, insbesondere einen Katheter, hineinzuziehen. Es ist in diesem Zusammenhang denkbar, dass ein weiterer, größerer Katheter vorgesehen ist, der im Gebrauch weiter proximal angeordnet ist. Der Führungsdraht 41, das Rekanalisationselement 10, das Konkrement 30 und das Zufuhrsystem 40 können in den größeren Katheter hineingeführt bzw. gezogen werden, um dann mit dem größeren Katheter vollständig aus dem Körper entfernt zu werden.

In Fig. 2 ist ein weiteres Ausführungsbeispiel der medizinischen Vorrichtung gezeigt, wobei das Rekanalisationselement 10 einen Hohlkörper 11 umfasst, der mehrere Windungen 13 bildet. Konkret weist das Rekanalisationselment 10 bzw. der Hohlköper 11 gemäß Fig. 2 fünf Windungen auf. Generell weist das Rekanalisationselement 10 im Wesentlichen eine spiralförmige Form bzw. Kontur auf. Eine andere Anzahl von Windungen ist möglich. Beispielsweise kann der Hohlkörper 11 höchstens 12, insbesondere höchstens 11, insbesondere höchstens 10, insbesondere höchstens 9, insbesondere höchstens 8, insbesondere höchstens 7, insbesondere höchstens 6, insbesondere höchstens 5, insbesondere höchstens 4, insbesondere höchstens 3,5, insbesondere höchstens 3, insbesondere höchstens 2,5, insbesondere höchstens 2, insbesondere höchstens 1,5, insbesondere höchstens 1 Windungen bilden. Eine höhere Anzahl an Windungen 13 ist möglich. Wie aus Fig. 2 ferner ersichtlich ist, weisen die Windungen 13 jeweils den gleichen Steigungswinkel bzw. die gleiche Steigung auf. Das schließt nicht aus, dass die Windungen 13 unterschiedliche Steigungen bzw. Steigungswinkel umfassen.

Der Steigungswinkel bzw. die Steigung der einzelnen Windungen, sind durch die physiologischen Gegebenheiten am Behandlungsort beeinflussbar. Beispielsweise kann eine Gefäßkrümmung oder die Form des Konkrements 30 dazu führen, dass die Steigungswinkel der einzelnen Windungen 13 variieren. Dasselbe gilt für den Windungsdurchmesser, also den Außendurchmesser des Rekanalisationselements 10. Das Rekanalisationselement 10 ist insgesamt flexibel und passt sich zweckmäßigerweise der Form des Konkrements 30 bzw. der Kontur des Körperhohlraums 20 an.

In Fig. 2 ist der Vorgang gezeigt, bei dem das Konkrement 30 aus dem Körperhohlraum 20 entfernt wird, wobei das Konkrement 30 beim Zurückziehen des Zufuhrsystems 40 durch größere Gefäßabschnitte geführt wird. Die Darstellung gemäß Fig. 2 mach deutlich, dass mit dem erfindungsgemäßen Rekanalisationselement 10 eine sichere Fixierung des Konkrements 30 erreicht wird, so dass das Konkrement 30 vollständig, insbesondere unter Vermeidung von Partikelablösung, aus dem Körperhohlraum 20, insbesondere einem Blutgefäß, entfernbar ist. Gleichzeitig erfolgt bereits während der Behandlung eine Rekanalisation des Körperhohlraums 20 durch den Hohlkörper 11. In Fig. 2 ist ferner gezeigt, dass der Hohlkörper 11 bzw. das Rekanalisationselement 20 proximal mit einem Führungsdraht 41 des Zufuhrsystems 40 verbunden ist. Der Führungsdraht 41 ermöglicht eine besonders einfach kontrollierbare Betätigung des Rekanalisationselements 10. Insbesondere ermöglicht der Führungsdraht 41 die Fixierung des Rekanalisationselements 10 im Bereich des Behandlungsortes, wobei durch Zurückziehen des Zufuhrsystems 40, insbesondere einer Katheterhülle, die Freisetzung des Rekanalisationselements 10 erfolgt. Dabei nimmt das Rekanalisationselement 10 den expandierten Zustand mit der spiralförmigen Struktur an.

Im Vergleich zu bekannten, sich geradlinig erstreckenden Systemen ermöglicht die medizinische Vorrichtung mit dem spiralförmig gewundenen Rekanalisationselement 10 eine Abdeckung bzw. Fixierung bzw. Sicherung des Konkrements 30 zu allen Seiten. Dadurch wird vermieden, dass sich das Konkrement 30 bzw. Konkrementbestandteile ablösen, wenn das Konkrement 30 beim Entfernen in größere Körperhohlgefäße gezogen wird. Insbesondere beim Passieren von Gefäßverzweigungen während der Entfernung des Konkrements 30 hält das spiralförmige Rekanalisationselement 10 das Konkrement 30 in einer festen Umklammerung, so dass ein unkontrolliertes Ablösen des Konkrements 30 bzw. von Konkrementpartikeln vermieden wird. Das spiralförmig gewundene Rekanalisationselement 10 bewirkt nicht nur einen Partikelablöseschutz in Umfangsrichtung bzw. zu allen Seiten des Konkrements 30, sondern ermöglicht auch, das Konkrement 30 radial zu komprimieren. Dazu erstreckt sich das Rekanalisationselement 10, insbesondere der Hohlkörper 11, radial außen um das Konkrement 30. Der Hohlkörper 11 umschlingt also durch die spiralförmige Windung 13 das Konkrement 30. Durch die Komprimierung werden das Ablösen und der Abtransport des Konkrements 30 erleichtert.

Fig. 3a zeigt durch entsprechende Strömungspfeile deutlich, wie die sofortige Rekanalisation eines Körperhohlraums 20 vorteilhaft erreicht wird. Durch den Hohlkörper 11 wird nach Freisetzen des Rekanalisationselements 10 eine Strömungspassage geschaffen, die im Wesentlichen spiralförmig um das Konkrement 30 führt. Bei der Thrombusbehandlung wird auf diese Weise schnell und sicher eine Blutströmung wiederhergestellt, so dass stromabwärts gelegene Gewebeareale schnell wieder mit Nährstoffen, insbesondere Sauerstoff, versorgt werden.

Bei dem Ausführungsbeispiel gemäß Fig. 3a bildet der Hohlkörper 11 mehrere, insbesondere fünf, Windungen 13, die einen relativ großen Steigungswinkel bzw. eine relativ große Steigung aufweisen. Im Unterschied zu einem Rekanalisationselement 10 mit einer kleineren Steigung der Windungen 13 (Fig. 3b) weist der Hohlkörper 11 bei dem Ausführungsbeispiel gemäß Fig. 3a im komprimierten Zustand eine relativ kleine Längserstreckung auf. Der Hohlkörper 11 des Rekanalisationselement 10 gemäß Fig. 3a ist also im komprimierten Zustand kürzer als der Hohlkörper 11 des Rekanalisationselements 10 gemäß Fig. 3b. Der im komprimierten Zustand kürzere Hohlkörper 11 erleichtert die Zuführung des Rekanalisationselements 10 durch das Zufuhrsystem 40, da die Schiebe- bzw. Zugkraft innerhalb des Zufuhrsystems 40 aufgrund einer geringeren Reibung reduziert ist. Auch im expandierten Zustand weist der Hohlkörper 11 gemäß Fig. 3a eine kleinere Kontaktfläche zum Körperhohlraum 20, insbesondere zu einer Gefäßwand, auf so dass eine Repositionierung des Rekanalisationselements 10 erleichtert ist. Überdies bildet der Hohlkörper 11 gemäß Fig. 3a eine relativ kurze Strömungspassage, so dass der Strömungswiderstand reduziert ist. Der Durchfluss durch das rekanalisierte Körpergefäß bzw. den rekanalisierten Körperhohlraum 20 wird somit erhöht.

Bei dem Ausführungsbeispiel gemäß Fig. 3b weist das Rekanalisationselement 10 eine vergleichsweise höhere Anzahl von Windungen 13 auf, wobei die Windungen 13 eine kleinere Steigung umfassen. Auf diese Weise wird der Kontakt zwischen dem Rekanalisationselement 10 und dem Konkrement 30 intensiviert, was eine verbesserte Fixierung des Konkrements 30 zur Folge hat. Überdies ermöglicht die Konfiguration gemäß Fig. 3b die Bereitstellung einer weiteren Strömungspassage, die nicht nur spiralförmig um das Konkrement 30 verläuft, sondern sich direkt zwischen der Gefäßwand des Körperhohlraums 20 und dem Konkrement 30 erstreckt. Durch die perforierte Struktur, insbesondere die geflochtene Gitterstruktur, des Hohlkörpers 11 bzw. der Wandung 12 wird ermöglicht, dass das Körperfluid im Wesentlichen geradlinig an dem Konkrement 30 vorbeiströmen kann. Der Hohlkörper 11 bildet also im Wesentlichen einen Abstandshalter zwischen der Gefäßwand des Körperhohlraums 20 und dem Konkrement 30. Der Strömungsquerschnitt im rekanalisierten Körperhohlraum 20 wird somit erweitert. Der Durchflusswiderstand wird dabei vermindert und somit der Rekanalisationsgrad bzw. der Durchfluss durch das rekanalisierte Gefäß bzw. den rekanalisierten Körperhohlraum 20 erhöht. Dies wird besonders vorteilhaft erreicht, wenn der Hohlkörper 11 eine Wandung 12 aufweist, die eine geflochtene Gitterstruktur umfasst. Das bedeutet, dass der Hohlkörper 11 bzw. allgemein das Rekanalisationselement 10 eine im Wesentlichen stentartige Struktur aufweist. Die Strömung des Körperfluids, insbesondere des Blutes, erfolgt dabei durch die Maschen der stentartigen Struktur.

Im Allgemeinen kann der Außendurchmesser des Rekanalisationselements 10, also der Außendurchmesser der Windungen 13, variieren. Beispielsweise kann das Rekanalisationselement 10 in einem distalen Abschnitt 17 eine distale Windung 13' aufweisen, die einen kleineren Außendurchmesser aufweist als die Windung 13 in einem proximalen Abschnitt 18. Das Rekanalisationselement 10 kann sich also - bezogen auf die spiralförmigen Windungen 13, 13' - verjüngen, wobei bevorzugt vorgesehen ist, dass sich das Rekanalisationselement 10 in distale Richtung, also zum distalen Abschnitt 17 hin, verjüngt. Dabei kann das Rekanalisationselement 10 insgesamt ein konusförmiges Profil bilden. Es ist auch möglich, dass das Rekanalisationselement 10 eine Spitze aufweist, die sich konusförmig verjüngt, wobei der Außendurchmesser der einzelnen Windungen 13' im distalen Abschnitt kleiner wird. Die Außenkontur des Rekanalisationselements 10 im proximalen Abschnitt 18 kann eine andere Form aufweisen. Beispielsweise kann die Außenkontur im proximalen Abschnitt 18 zylinderförmig gestaltet sein. Durch den verjüngten distalen Abschnitt 17 wird vermieden, dass Konkrementpartikel, die sich möglicherweise vom Konkrement 30 ablösen, frei in den Körperhohlraum 20 gelangen und durch das Körperfluid, insbesondere Blut, in stromabwärts gelegene Areale geschwemmt werden. Das konusförmig gestaltete Ende des Rekanalisationselements 10 bildet also im Wesentlichen eine Filterfunktion.

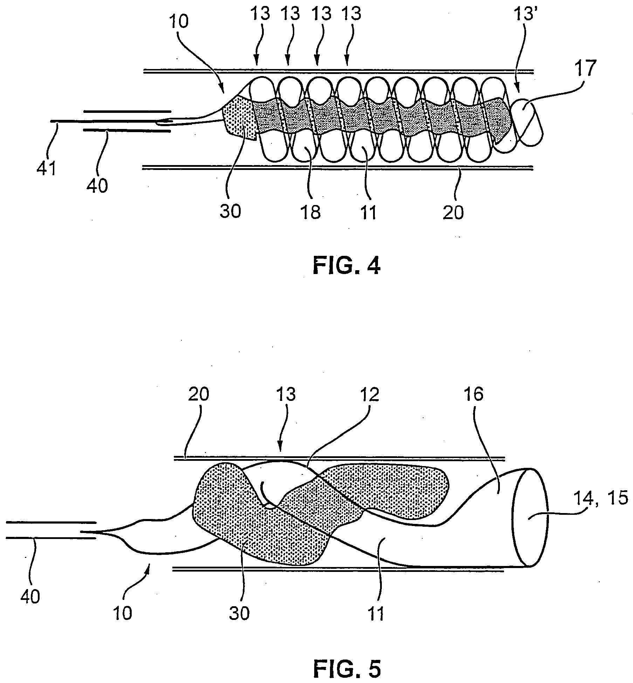

Eine zusätzliche oder alternative Möglichkeit zur Bereitstellung einer Filterfunktion am distalen Ende 14 des Rekanalisationselements 10, ist in Fig. 5 dargestellt. Fig. 5 zeigt ein Ausführungsbeispiel der medizinischen Vorrichtung, die im Wesentlichen einen ähnlichen Aufbau wie das Ausführungsbeispiel gemäß Fig. 1 aufweist. Der Unterschied besteht darin, dass bei dem Rekanalisationselement gemäß Fig. 5 der Hohlkörper 11 am distalen Ende 14 einen Filterabschnitt 16 bildet. Dazu weist der Hohlkörper 11 am distalen Ende 14 einen vergrößerten Querschnittsdurchmesser auf. Der Querschnittsdurchmesser des Hohlkörpers 11 ist am distalen Ende 14 größer als in einem proximalen Abschnitt 18 des Rekanalisationselements 10. Der Hohlkörper 11 gemäß Fig. 5 bildet also am distalen Ende 14 eine Aufweitung. Auf diese Weise überdeckt der Hohlkörper 11 am distalen Ende 14 die gesamte Querschnittsfläche des Körperhohlraums 20 und fungiert als Filter. Durch die perforierte Struktur der Wandung 12 des Hohlkörpers 11, insbesondere durch die geflochtene Gitterstruktur, wird weiterhin gewährleistet, dass eine effiziente Rekanalisation des Körperhohlraums 20 erfolgt.

Bei einem weiteren Ausführungsbeispiel, das in Fig. 6 dargestellt ist, verjüngt sich das distale Ende 14 des Hohlkörpers 11. Der Hohlkörper 11 bildet also eine distale Spitze, die im Wesentlichen konusförmig geformt sein kann. Das Rekanalisationselement 10 weist somit insgesamt im Wesentlichen eine korkenzierartige Kontur auf. Durch die distale Spitze bzw. das sich verjüngende distale Ende 14 des Hohlkörpers 11 passt sich das Rekanalisationselement 10 bei Vorschieben in kleinere Gefäße besser der Gefäßkontur an. Das distale Ende 14 des Hohlkörpers 11 ist ferner mit einem Betätigungselement 19 gekoppelt, das parallel zum Führungsdraht 41 durch das Zufuhrsystem 40 verläuft. Das Betätigungselement 19 ermöglicht die im Wesentlichen axiale Betätigung des Rekanalisationselements 10. Das bedeutet, dass beispielsweise durch Ziehen des Betätigungselements 19 in proximale Richtung das Rekanalisationselement 10 axial komprimiert wird. Die Steigung der spiralförmigen Windungen 13 wird somit reduziert. Alternativ kann durch eine Bewegung des Betätigungselements 19 in distale Richtung das Rekanalisationselement 10 gestreckt werden. Durch die Streckung wird der Außendurchmesser des Rekanalisationselements 10 bzw. der spiralförmigen Windung 13 reduziert, so dass das Konkrement 30 weiter fixiert wird. Zusätzlich wird der Abstand zwischen dem Außenumfang des Rekanalisationselements 10 und der Gefäßwand erhöht, so dass die Reibung des Rekanalisationselements 10 an der Gefäßwand beim Zurückziehen reduziert oder aufgehoben wird. Die Gefäßwand bzw. der Körperhohlraum 20 wird somit geschont.

Der Hohlkörper 11 bzw. die Wandung 12 können eine vollständige, insbesondere geschlossene, Abdeckung 12a aufweisen. Das bedeutet, dass bei dem Ausführungsbeispiel gemäß Fig. 6 die Wandung 12 beispielsweise vollständig mit einer fluiddichten Abdeckung 12a überzogen ist. Die Wandung 12 kann auch selbst fluiddicht ausgebildet sein. Die für die Rekanalisation erforderliche Strömungspassage bzw. Rekanalisationspassage wird in diesem Fall dadurch erreicht, dass die spiralförmigen Windungen 13 bzw. allgemein der Hohlkörper 11 das Konkrement 30 vollständig spiralförmig umschlingt und komprimiert. Dadurch wird eine Strömungspassage eröffnet, die sich zwischen dem Außenumfang des Konkrements bzw. dem Außenumfang, insbesondere der Außenkontur, des Rekanalisationselements 10 und der Gefäßwand des Körperhohlraums 20 erstreckt. Zwischen dem Konkrement 30 bzw. der Außenkontur des Rekanalisationselements 10 und der Gefäßwand des Körperhohlraums 20 wird also ein Strömungsspalt, insbesondere ein Ringspalt, gebildet, der eine Fluidströmung an dem Konkrement 30 vorbei ermöglicht.

Das Betätigungselement 19 kann generell durch das Rekanalisationselement 10, insbesondere durch die spiralförmigen Windungen 13 verlaufen. Alternativ kann das Betätigungselement 19 entlang einer Außenkontur des Rekanalisationselements 10 bzw. der spiralförmigen Windungen 13 verlaufen.

Bei einer weiteren Variante gemäß Fig. 7 ist vorgesehen, dass der Hohlkörper 11 bzw. die Wandung 12 zumindest teilweise mit einer Abdeckung 12a versehen ist bzw. mit einer Abdeckung 12a bespannt ist. Vorzugsweise ist der Hohlkörper 11 vollständig mit der Abdeckung 12a versehen. Die Abdeckung 12a kann Saugöffnungen 12b umfassen, die vorzugsweise in Richtung der zentralen Achse des spiralförmig gewundenen Rekanalisationselements 10 gerichtet bzw. im Gebrauch dem Konkrement 30 zugewandt sind. Die medizinische Vorrichtung ist in diesem Falle vorzugsweise mit einer Aspirationseinheit (nicht dargestellt) gekoppelt, so dass in dem Hohlkörper 11 ein Unterdruck erzeugbar ist. Durch die Saugöffnungen wird dabei erreicht, dass das Konkrement 30 durch das Rekanalisationselement 10 angesaugt wird. Die feste Verbindung zwischen dem Rekanalisationselement 10 und dem Konkrement 30 wird somit gestärkt. Vorzugsweise ist die Wandung 12, insbesondere bei Verwendung einer Aspirationseinheit im Bereich des distalen Abschnitts 17 bzw. des proximalen Abschnitts 18 vollständig mit der Abdeckung 12a bespannt. Auf diese Weise wird verhindert, dass durch den erzeugten Unterdruck Körperfluid, insbesondere Blut, angesaugt wird. Der Unterdruck kann daher die maximale Leistung erreichen.

Um den Unterdruck, der vorzugsweise außerhalb des Körpers erzeugt wird, über das Zufuhrsystem 40 in den Hohlkörper 11 einzuleiten, kann im proximalen Abschnitt 18 des Rekanalisationselements 10 eine Öffnung vorgesehen sein, die im Gebrauch innerhalb des Zufuhrsystems 40 angeordnet ist und eine Fluidverbindung zwischen dem Zufuhrsystem 40 und dem Hohlkörper 11 bereitstellt.

Es ist denkbar, dass der proximale Abschnitt 18 und/oder distale Abschnitt 17 partiell mit der Abdeckung 12a versehen sind. Auf diese Weise wird ermöglicht, dass höhere absolute Unterdruckwerte erreichbar sind. Durch die Öffnungen, insbesondere Saugöffnungen 12b, in der Abdeckung 12a können auch Medikamente an den Behandlungsort geleitet werden.

Für die geflochtene Gitterstruktur sind die folgenden Konstruktionsmerkmale vorteilhaft:
Zur Verwendung der medizinischen Vorrichtung in Körperhohlräumen, insbesondere Blutgefäßen, mit einem Gefäßdurchmesser von 2 mm bis 3 mm weist das Rekanalisationselement 10, insbesondere die spiralförmige Windung 13, vorzugsweise einen Außendurchmesser im Bereich von 2 mm bis 8 mm, insbesondere 3 mm bis 6mm, insbesondere 3 mm bis 5 mm. Dabei umfasst der Hohlkörper 11 vorzugsweise einen Querschnittsdurchmesser im Bereich von 1 mm bis 2,5 mm, insbesondere 1,5 mm bis 2,5 mm, insbesondere 1,8 mm bis 2,2 mm.

Für einen Gefäßdurchmesser bzw. Durchmesser des Körperhohlraums 20 von 3 mm bis 4 mm hat sich ein Außendurchmesser des Rekanalisationselements 10 bzw. der spiralförmigen Windung 13 im Bereich von 3 mm bis 10 mm, insbesondere 4 mm bis 8 mm, insbesondere 4 mm bis 6 mm, als vorteilhaft erwiesen. Der Hohlkörper 11 kann in diesem Fall einen Querschnittsdurchmesser im Bereich von 1 mm bis 3 mm, insbesondere 1,5 mm bis 2,5 mm, insbesondere 2,0 mm bis 2,5 mm aufweisen.

Wenn die medizinische Vorrichtung in Körperhohlräume 20 mit einem Querschnittsdurchmesser von 4 mm bis 5 mm eingesetzt wird, ist es vorteilhaft, wenn das Rekanalisationselement 10 bzw. die Windung 13 einen Außendurchmesser aufweist, der sich von 4 mm bis 12 mm, insbesondere von 5 mm bis 10 mm, insbesondere von 5 mm bis 7 mm, erstreckt. Der Hohlkörper 11 weist dabei vorzugsweise einen Querschnittsdurchmesser im Bereich von 1,5 mm bis 4 mm, insbesondere 2 mm bis 3 mm, insbesondere 2,5 mm bis 3 mm auf.

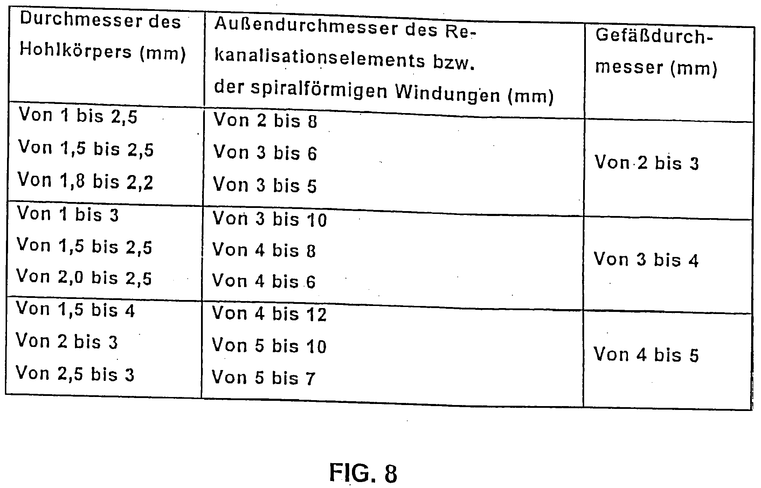

Weitere bevorzugte Werte für den Durchmesser des Hohlkörpers 11 bzw. den Außendurchmesser des Rekanalisationselements 10 in Relation zum Durchmesser des Körperhohlraums 20 sind in der Tabelle gemäß Fig. 8 dargestellt. Für weitere Gefäßdurchmesser lassen sich die entsprechenden Dimensionen der medizinischen Vorrichtung berechnen.

Grundsätzlich kann der Außendurchmesser des Rekanalisationselements 10 bzw. der spiralförmigen Windung 13 im expandierten Zustand etwas größer sein als der Querschnittsdurchmesser des Zielgefäßes bzw. Körperhohlraums 20. Damit wird bewirkt, dass sich das Rekanalisationselement 10 fest an die Gefäßwand anlegt. Es ist auch möglich, dass der Außendurchmesser des Rekanalisationselements 10 kleiner als das Lumen bzw. der Durchmesser des Zielgefäßes ist. In diesem Falle wird erreicht, dass das Rekanalisationselement 10 die Gefäßwand im Wesentlichen nicht berührt. Dadurch wird eine weitere Strömungspassage geschaffen, die zwischen der Gefäßwand und dem Außenumfang des Rekanalisationselements 10 ausgebildet ist. Vorteilhaft ist dabei, dass einerseits der Widerstand für den Durchfluss des Körperfluids, insbesondere der Strömungswiderstand für fließendes Blut, weiter reduziert, der Thrombus bzw. allgemein das Konkrement 30 fester fixiert und andererseits die Gefäßwand des Körperhohlraums 20 geschont wird, wenn das Rekanalisationselement 10 in proximaler Richtung aus dem Gefäß gezogen wird.

Bei den vorgenannten Konfigurationen entspricht der Querschnittsdurchmesser des Hohlkörpers 11 im Wesentlichen dem Querschnittsdurchmesser des Gittergeflechts bzw. der geflochtenen Gitterstruktur, die die Wandung 12 des Hohlkörpers 11 bildet. Besonders bevorzugt ist es, wenn die geflochtene Gitterstruktur acht fadenförmige Elemente umfasst, wobei die fadenförmige Elemente, insbesondere Drähte, vorzugsweise einen Querschnittsdurchmesser im Bereich von 40 µm bis 76 µm, insbesondere 50 µm bis 68 µm, umfasst. Es können auch 6 bis 12 fadenförmige Elemente vorgesehen sein, die einen Querschnittsdurchmesser im Bereich von 50 µm bis 68 µm aufweisen. Ferner ist es möglich, dass 6 bis 18 fadenförmige Elemente vorgesehen sind, die einen Querschnittsdurchmesser im Bereich von 35 µm bis 76 µm aufweisen.

Im Allgemeinen können unterschiedliche fadenförmige Elemente, insbesondere Drähte, zur Bildung der geflochtenen Gitterstruktur kombiniert werden. Beispielsweise können insgesamt 16 Drähte vorgesehen sein, wobei 8 Drähte einen Querschnittsdurchmesser von 76 µm und weitere 8 Drähte einen Querschnittsdurchmesser von 40 µm aufweisen. Alternativ kann eine erste Hälfte der fadenförmigen Elemente einen Querschnittsdurchmesser von 68 µm und eine zweite Hälfte der fadenförmigen Elemente einen Querschnittsdurchmesser von 50 µm aufweisen. Es ist auch möglich, dass die geflochtene Gitterstruktur aus 6 fadenförmigen Elementen, die einen Querschnittsdurchmesser von 68 µm aufweisen, und weiteren 12 fadenförmigen Elementen bzw. Drähten mit einem Querschnittsdurchmesser von 50 µm gebildet ist. Eine weitere Alternative sieht vor, die geflochtene Gitterstruktur aus 6 Drähten mit einem Querschnittsdurchmesser von 76 µm und weiteren 18 Drähten mit einem Querschnittsdurchmesser von 35 µm auszubilden.

Prinzipiell sind alle Kombinationen aus unterschiedlichen Drähten möglich. In einer bevorzugten Konfiguration umfasst das Rekanalisationselement 10 eine geflochtene Gitterstruktur, die aus gleichen Drähten gebildet ist, wobei die Drahtanzahl 16, 20 oder 24 Drähte betragen kann, die jeweils einen Durchmesser von 50 µm, 63 µm, 69 µm oder 76 µm aufweisen können. Alle weiteren Kombinationen aus Drahtanzahl und Drahtstärke sind möglich, wobei innerhalb des Rekanalisationselements 10 auch unterschiedlichen Drahtstärken vorgesehen sein können.

Die Anzahl der fadenförmigen Elemente bzw. Drähte wird dabei durch einen senkrechten Querschnitt durch den Hohlkörper 11 ermittelt. Die Drahtanzahl bezieht sich also auf die Anzahl der Drähte, die durch eine zum rohrförmigen Geflecht, insbesondere zum Hohlkörper 11 bzw. zur spiralförmig gewundenen Mittelachse des Hohlkörpers 11, senkrechten Ebene verlaufen.

Es ist besonders vorteilhaft, wenn die fadenförmigen Elemente, insbesondere Drähte, an einem Ende der geflochtenen Gitterstruktur, insbesondere am distalen Ende 14 des Rekanalisationselements 10 geschlossene Schlaufen bilden. Die Drähte werden also am distalen Ende 14 umgelenkt und durch die Gitterstruktur des Hohlkörpers 11 zurückgeführt. Auf diese Weise wird erreicht, dass die Verletzungsgefahr für die Gefäßwand eines Körperhohlraums 20 reduziert wird. Es wird also ein atraumatisches Geflechtende gebildet. Es ist auch möglich, dass die fadenförmigen Elemente am distalen Ende 14 zur Bildung der Schlaufen miteinander verbunden sind, beispielsweise durch eine Hülse. Dabei sind die Verbindungen der drahtförmigen Elemente vorzugsweise in der Umfangsebene der geflochtenen Gitterstruktur des Hohlkörpers 11 angeordnet.

Vorzugsweise umfasst der Hohlkörper 11, insbesondere die geflochtene Gitterstruktur, ein selbstexpandierendes Material, beispielsweise eine Nickel-Titan-Legierung. Das Rekanalisationselement 10 kann auch einen selbstexpandierenden Kunststoff aufweisen. Generell können die selbstexpandierenden Eigenschaften durch einen Formgedächtniswerkstoff, insbesondere ein Formgedächtnismetall oder einen Formgedächtniskunststoff, erreicht werden.

Der Hohlkörper 11 bzw. allgemein das Rekanalisationselement 10 kann durch Laserschneiden aus einem Rohrmaterial oder Flachmaterial hergestellt sein. Bei der Verwendung eines Flachmaterials wird dieses anschließend in eine Rohrform gebogen und an den sich berührenden Längskanten verbunden, beispielsweise verschweißt oder verlötet. Alternativ kann der Hohlkörper 11 bzw. das Rekanalisationselement 10 durch eine Dünnschichttechnik, insbesondere ein Sputterverfahren, hergestellt werden. Besonders bevorzugt ist es, wenn der Hohlkörper 11 bzw. das Rekanalisationselement 10 durch Flechten von Drähten hergestellt wird, wobei die Drähte sowohl Metalldrähte, als auch Kunststoffdrähte oder eine Kombination davon umfassen können. Die Herstellung des Rekanalisationselements 10 erfolgt vorzugsweise in einer teilweise expandierten Form. Vorzugsweise wird der Hohlkörper 11 in einer geraden, zylindrischen Form geflochten - bedarfsweise mit sich aufweitenden oder verjüngenden Endabschnitten (proximale Trichterform bzw. distaler Filterabschnitt) - und auf einem spiralförmigen Dorn einer Wärmebehandlung unterzogen, so dass sich die spiralförmige Windung 13 einstellt. Das endgefertigte Rekanalisationselement 10 wird anschließend komprimiert bzw. gecrimpt und in ein Zufuhrsystem 40 geladen. Das Zufuhrsystem 40 umfasst vorzugsweise einen medizinischen Katheter und kann zusätzlich ein Zufuhrelement, beispielsweise den Führungsdraht 41, aufweisen. Es ist auch möglich, dass die Zufuhr des Rekanalisationselements 10 durch den Katheter pneumatisch oder hydraulisch erfolgt. Über den Katheter können ferner medizinische Wirkstoffe, insbesondere antithrombogene Medikamente, an den Behandlungsort geführt werden.

Die erfindungsgemäße medizinische Vorrichtung eignet sich besonders zur Rekanalisation von Blutgefäßen, die durch einen Thrombus verschlossen sind. Dabei kann sowohl ein partieller, als auch vollständiger Verschluss vorliegen. Bevorzugt ist der Einsatz der medizinischen Vorrichtung zur Rekanalisation von Herzkranzgefäßen, zerebralen Gefäßen, Pulmonalarterien oder Karotisarterien. Weitere Anwendungsmöglichkeiten sind beispielsweise die Rekanalisation von Strömungskanälen der Harnwege, beispielsweise des Harnleiters.

Die Erfindung ist nicht auf eine medizinische Vorrichtung zum Rekanalisieren von Körperhohlräumen beschränkt. Vielmehr wird allgemein eine medizinische Vorrichtung mit einem in einen Körperhohlraum einführbaren Hohlkörper 11 offenbart, der von einem komprimierten Zustand mit einem ersten Querschnittsdurchmesser in einen expandierten Zustand mit einem zweiten Querschnittsdurchmesser überführbar ist, der größer als der erste Querschnittsdurchmesser ist. Im expandierten Zustand ist der Hohlkörper 11 spiralförmig gewunden bzw. bildet einen spiralförmigen Hohlkanal. Insofern entspricht der Hohlkörper dem vorstehend beschriebenen Hohlkörper. Andere Anwendungsmöglichkeiten als die Rekanalisierung von Körperhohlräumen sind mit dem spiralförmig gewundenen Hohlkörper, der selbst wiederum expandierbar ist, möglich. Mit anderen Worten wird ein stentähnlicher Hohlkörper mit spiralförmig gewundener Außenkontur bzw. ein spiralförmig gewundener Stent offenbart .

Der Vorteil des zu einem Hohlkanal expandierbaren Hohlkörpers 11 mit spiralfömiger Außenkontur besteht darin, dass durch Streckung der Spiralform eine Verlängerung des Implantats herbeigeführt werden kann, ohne dass dabei das Lumen bzw. der Querschnittsdurchmesser des Hohlkörpers 11 verringert wird im Gegensatz zu einem nicht-spiralförmigen, zylindrischen Stent. Dies ermöglicht die Fixierung des Hohlkörpers an den Enden in an sich bekannter Weise durch Expansion im Gefäß. Der mittlere Bereich des Hohlkörpers kann frei beweglich sein und folgt aufgrund der Verlängerung durch Streckung der Spiralform der Gefäßdehnung, die durch die Pulsation auftritt. Dabei tritt keine Verringerung des Lumens des Hohlkörpers 11 auf. Damit eignet sich das Implantat zur Behandlung von Aneurysmen (Fusiformenaneurysmen).

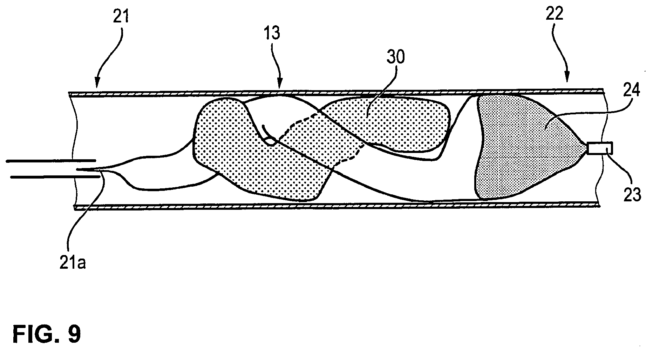

Bei einer bevorzugten Ausgestaltung der medizinischen Vorrichtung kann ein distales Ende 22 des Hohlkörpers 11 fluidicht verschlossen sein, insbesondere derart, dass ein in den Hohlkörper 11 eingebrachtes Medikament im implantierten Zustand im Bereich des Hohlkörpers 11 zurückgehalten wird. Ein Beispiel hierzu wird anhand von Fig. 9 erläutert.

Darin ist ein spiralförmiger expandierbarer Hohlkörper 11 ähnlich wie der Hohlkörper 11 gemäß Fig . 5 gezeigt. Der Hohlkörper 11 weist eine Wandung 12 aus einer Gitterstruktur, insbesondere aus einem Gittergeflecht auf. Das distale Ende 22 ist fluiddicht, insbesondere fluidicht und fest, verschlossen. Hierfür kann sich beispielsweise das distale Ende 22 in proximaler Richtung verjüngen, so dass ein distaler Verschluss gebildet ist. Konkret können die Gitterelemente, insbesondere die Drahtelemente nach innen geführt und an der Spitze des distalen Endes 22 verbunden sein. Hierfür kann eine Hülse 23 vorgesehen sein, in der die freien Enden der Drahtelemente gehalten sind. Die Drahtelemente können auch anderweitig fixiert sein, bspw. durch Verdrillen.

Das distale Ende 22 ist mit einer fluidichten Abdeckung 24 fest verbunden. Zweck der Abichtung ist es, Medikamente, bspw. Thromben lösende Mittel im Bereich proximal vor der Abdeckung zurückzuhalten, damit diese auf einen Thrombus einwirken können, der von dem spiralförmigen Hohlkörper 11 umschlungen ist, wie in Fig. 9 gezeigt. Für den dafür erforderlichen Verschluss des Gefäßes ist das distale Ende 22 aufgeweitet, insbesondere trompetenförmig aufgeweitet. Der Durchmesser des distalen Endes 22 ist größer als der Durchmesser des Lumens des Hohlkörpers 11 im Bereich der Windung 13. Der Durchmesser des distalen Endes 22 ist mindestens so groß wie der Außendurchmesser der Spirale. Damit wird erreicht, dass sich das distale Ende 22 an die Gefäßwand im Bereich der Abdeckung anlegt und diese abdichtet. Für den Fall, dass ein sich stark verjüngendes Gefäß abgedichtet werden soll, kann der Durchmesser des distalen Endes 22 auch kleiner als der Außendurchmesser der Spirale sein.

Die Einwirkung des Medikaments auf den gefangenen Thrombus wird durch die unbedeckte Gitterstruktur des Hohlkörpers 11 im Bereich der Windung 13 erreicht. Die Abdeckung 24 endet etwas vor der Windung 13 und wirkt wie eine Kappe.

Der Hohlkörper 11 kann fest oder lösbar mit dem Führungsdraht verbunden sein.

Die vorstehend genannten Merkmale des distal verschlossenen Hohlkörpers werden sowohl in Kombination als auch einzeln im Zusammenhang mit den anderen Beispielen bzw. allgemein im Zusammenhang mit der Erfindung offenbart. Beispielsweise können mehr als eine Windung 13 vorgesehen sein.

Das Verfahren zur Herstellung eines derartigen Hohlkörpers bzw. einer derartigen medizinischen Vorrichtung umfasst den Schritt, wonach der Hohlkörper auf einen spiralförmigen Dorn aufgeschoben und wärmebehandelt wird, sodass dem Hohlkörper eine Spiralform aufgeprägt wird, die sich bei entsprechender Umgebungstemperatur im Gebrauch einstellt. Der Hohlkörper ist dementsprechend aus einem Formgedächtswerkstoff hergestellt und selbst-expandierbar. Die Gitterstruktur des Hohlkörpers kann in an sich bekannter Weise hergestellt sein, beispielsweise durch Flechten oder Schneiden oder durch ein Abscheideverfahren.

### Bezugszeichenliste

- 10: Rekanalisationselement
- 11: Hohlkörper
- 12: Wandung
- 12a: Abdeckung
- 12b: Saugöffnung
- 13: Windung
- 13': distale Windung
- 14: distales Ende
- 15: axiale Öffnung
- 16: Filterabschnitt
- 17: distaler Abschnitt
- 18: proximaler Abschnitt
- 19: Betätigungselement
- 20: Körperhohlraum
- 21: proximales Ende
- 21a: Übergangsbereich
- 22: distales Ende
- 23: Hülse
- 24: Abdeckung
- 30: Konkrement
- 40: Zufuhrsystem
- 41: Führungsdraht

## Patentansprüche

1. Medizinische Vorrichtung zum Rekanalisieren von Körperhohlräumen (20) mit einem in den Körperhohlraum (20) einführbaren Rekanalisationselement (10), das von einem komprimierten Zustand in einen expandierten Zustand überführbar ist, wobei die Außenkontur des Rekanalisationselements (10) im expandierten Zustand wenigstens eine spiralförmige Windung (13) bildet, und wobei das Rekanalisationselement (10) im expandierten Zustand einen rohrförmigen Hohlkörper (11) aufweist derart, dass der Körperhohlraum (20) rekanalisierbar ist,
**dadurch gekennzeichnet, dass**
der Hohlkörper (11) von einem komprimierten Zustand in einen expandierten Zustand überführbar ist und im expandierten Zustand einen Hohlkanal bildet, der durch eine Aufweitung des Hohlkörpers (11) während der Expansion des Rekanalisationselements (10) entsteht.

2. Medizinische Vorrichtung nach Anspruch 1,
**dadurch gekennzeichnet, dass**
der Hohlkörper (11) durch eine rohrförmige Wandung (12) begrenzt ist, die zumindest in einem proximalen Abschnitt (18) und/oder in einem distalen Abschnitt (17) mindestens eine Öffnung aufweist.

3. Medizinische Vorrichtung nach Anspruch 2,
**dadurch gekennzeichnet, dass**
die Wandung (12) zumindest abschnittsweise eine perforierte Struktur und/oder eine geflochtene Gitterstruktur aufweist.

4. Medizinische Vorrichtung nach Anspruch 2 oder 3,
**dadurch gekennzeichnet, dass**
die Wandung (12), insbesondere die perforierte Struktur, ein lasergeschnittenes oder durch Dünnschichttechnik hergestelltes, insbesondere gesputtertes, Strukturelement umfasst.

5. Medizinische Vorrichtung nach Anspruch 3,
**dadurch gekennzeichnet, dass**
die geflochtene Gitterstruktur fadenförmige Elemente umfasst, die an einem distalen Ende der Gitterstruktur geschlossene Schlaufen bilden.

6. Medizinische Vorrichtung nach wenigstens einem der Ansprüche 1 bis 5,
**dadurch gekennzeichnet, dass**
der Hohlkörper (11) im expandierten Zustand zumindest abschnittsweise unterschiedliche Querschnittsdurchmesser, insbesondere an einem distalen Ende (14) einen größeren Querschnittsdurchmesser als in einem proximalen Abschnitt (18), aufweist.

7. Medizinische Vorrichtung nach wenigstens einem der Ansprüche 1 bis 6,
**dadurch gekennzeichnet, dass**
der Hohlkörper (11) im expandierten Zustand an einem distalen Ende (14) des Rekanalisationselements (10) einen Filterabschnitt (16) bildet.

8. Medizinische Vorrichtung nach wenigstens einem der Ansprüche 1 bis 7,
**dadurch gekennzeichnet, dass**
die spiralförmige Windung (13) in einem distalen Abschnitt (17) des Rekanalisationselements einen kleineren Windungsdurchmesser als in einem proximalen Abschnitt (18) des Rekanalisationselements (10) aufweist.

9. Medizinische Vorrichtung nach wenigstens einem der Ansprüche 1 bis 8,
**dadurch gekennzeichnet, dass**
das Rekanalisationselement (10) proximal mit einem Zufuhrelement, insbesondere einem Führungsdraht (41), verbunden oder verbindbar ist.

10. Medizinische Vorrichtung nach wenigstens einem der Ansprüche 1 bis 9,
**dadurch gekennzeichnet, dass**
das Rekanalisationselement (10) eine einen Wirkstoff freisetzende, insbesondere antithrombogene, Beschichtung aufweist.

11. Medizinische Vorrichtung nach wenigstens einem der Ansprüche 1 bis 10,
**dadurch gekennzeichnet, dass**
das Rekanalisationselement (10), insbesondere der Hohlkörper (11), wenigstens abschnittsweise eine fluiddichte Abdeckung (12a) aufweist, die insbesondere ein oder mehrere Saugöffnungen (12b) umfasst, die im Gebrauch einem Konkrement (30) zugewandt sind.

12. Medizinische Vorrichtung nach wenigstens einem der Ansprüche 1 bis 11,
**dadurch gekennzeichnet, dass**
das Rekanalisationselement (10) an einem distalen Ende (14) mit einem Betätigungselement (19) verbunden ist, das sich im Wesentlichen längsaxial entlang des Rekanalisationselements (10) erstreckt.

13. Medizinische Vorrichtung nach Anspruch 12,
**dadurch gekennzeichnet, dass**
das Betätigungselement (19) zumindest teilweise entlang einer Außenkontur und/oder einer Innenkontur des Rekanalisationselements (10) verläuft.

14. Set umfassend eine medizinische Vorrichtung nach Anspruch 1 und ein Zufuhrsystem (40) für eine derartige Vorrichtung.

15. Verfahren zur Herstellung einer medizinischen Vorrichtung nach einem der vorhergehenden Ansprüche, bei dem ein expandierbarer und komprimierbarer Hohlkörper (11) aus einem Formgedächtsniswerkstoff auf einem spiralförmigen Dorn aufgeschoben und wärmebehandelt wird, so dass dem Hohlkörper (11) eine Spiralform aufgeprägt wird.

## Claims

1. A medical device for the recanalisation of body cavities (20), comprising a recanalisation element (10), which can be inserted into the body cavity (20) and which can be transferred from a compressed state into an expanded state, wherein the outer contour of the recanalisation element (10) in the expanded state forms at least one helical winding (13), and wherein the recanalisation element (10) in the expanded state has a tubular hollow body (11), such that the body cavity (20) can be recanalised,
**characterised in that**
the hollow body (11) can be transferred from a compressed state into an expanded state, and in the expanded state forms a hollow channel, which is created by widening the hollow body (11) during the expansion of the recanalisation element (10).

2. The medical device according to claim 1,
**characterised in that**
the hollow body (11) is delimited by a tubular wall (12), which has at least one opening at least in a proximal portion (18) and/or in a distal portion (17).

3. The medical device according to claim 2,
**characterised in that**
the wall (12) has a perforated structure and/or a braided lattice structure at least in portions.

4. The medical device according to claim 2 or 3, **characterised in that**
the wall (12), in particular the perforated structure, comprises a laser-cut structural element or a structural element that has been produced by thin-film technology, in particular a sputtered structural element.

5. The medical device according to claim 3,
**characterised in that**
the braided lattice structure comprises thread-like elements, which form closed loops at a distal end of the lattice structure.

6. The medical device according to at least one of claims 1 to 5,
**characterised in that**
the hollow body (11) in the expanded state has different cross-sectional diameters at least in portions, in particular has a cross-sectional diameter at a distal end (14) greater than in a proximal portion (18).

7. The medical device according to at least one of claims 1 to 6,
**characterised in that**
the hollow body (11) in the expanded state forms a filter portion (16) at a distal end (14) of the recanalisation element (10).

8. The medical device according to at least one of claims 1 to 7,
**characterised in that**
the helical winding (13) in a distal portion (17) of the recanalisation element has a winding diameter smaller than in a proximal portion (18) the recanalisation element (10).

9. The medical device according to at least one of claims 1 to 8,
**characterised in that**
the recanalisation element (10) is connected or can be connected proximally to a feed element, in particular a guide wire (41).

10. The medical device according to at least one of claims 1 to 9,
**characterised in that**
the recanalisation element (10) has an active-substance-releasing, in particular antithrombogenic coating.

11. The medical device according to at least one of claims 1 to 10,
**characterised in that**
the recanalisation element (10), in particular the hollow body (11), has a fluid-tight covering (12a) at least in portions, which in particular comprises one or more suction openings (12b), which face toward a concretion (30) during use.

12. The medical device according to at least one of claims 1 to 11,
**characterised in that**
the recanalisation element (10) is connected at a distal end (14) to an actuation element (19) extending substantially longitudinally axially along the recanalisation element (10).

13. The medical device according to claim 12,
**characterised in that**
the actuation element (19) extends at least in part along an outer contour and/or an inner contour of the recanalisation element (10).

14. A set comprising a medical device according to claim 1 and a feed system (40) for a device of this type.

15. A method for producing a medical device according to any one of the preceding claims, in which an expandable and compressible hollow body (11) made of a shape-memory material is slid onto a helical mandrel and is heat-treated so that a spiral shape is impressed onto the hollow body (11).

## Revendications

1. Dispositif médical de recanalisation d'organes tubulaires (20) comportant un élément de recanalisation (10) pouvant être introduit dans l'organe tubulaire (20), qui peut être passé d'un état comprimé à un état expansé, le contour extérieur de l'élément de recanalisation (10) constituant en état expansé au moins une spire en forme de spirale (13) et l'élément de recanalisation (10) présentant en état expansé un corps creux de forme tubulaire (11) de manière à pouvoir recanaliser l'organe tubulaire (20),
**caractérisé en ce que**
le corps creux (11) peut être passé d'un état comprimé à un état expansé et constitue en état expansé un canal creux qui apparaît du fait d'un élargissement du corps creux (11) pendant l'expansion de l'élément de recanalisation (10).

2. Dispositif médical selon la revendication 1,
**caractérisé en ce que**
le corps creux (11) est limité par une paroi de forme tubulaire (12) qui présente une ouverture au moins dans une section proximale (18) et/ou dans une section distale (17).

3. Dispositif médical selon la revendication 2,
**caractérisé en ce que**
la paroi (12) présente du moins par endroits une structure perforée et/ou une structure grillagée tressée.

4. Dispositif médical selon la revendication 2 ou 3,
**caractérisé en ce que**
la paroi (12), en particulier la structure perforée, comprend un élément structurel découpé au laser ou fabriquais par technologie des couches minces, en particulier par pulvérisation.

5. Dispositif médical selon la revendication 3,
**caractérisé en ce que**
la structure grillagée tressée comprend des éléments en forme de fils qui constituent des boucles fermées à une extrémité distale de la structure grillagée.

6. Dispositif médical selon au moins une des revendications 1 à 5,
**caractérisé en ce que**
le corps creux (11) présente en état expansé, du moins par endroits, différents diamètres de section transversale, en particulier à une extrémité distale (14) un diamètre de section transversale plus grand que dans une extrémité proximale (18).

7. Dispositif médical selon au moins une des revendications 1 à 6,
**caractérisé en ce que**
le corps creux (11) constitue en état expansé une section de filtre (16) à une extrémité distale (14) de l'élément de recanalisation (10).

8. Dispositif médical selon au moins une des revendications 1 à 7,
**caractérisé en ce que**
la spire en forme de spirale (13) présente, dans une section distale (17) de l'élément de recanalisation, un diamètre de spire inférieur à un diamètre dans une section proximale (18) de l'élément de recanalisation (10).

9. Dispositif médical selon au moins une des revendications 1 à 8,
**caractérisé en ce que**
l'élément de recanalisation (10) est raccordé ou raccordable au niveau proximal avec un élément d'acheminement, en particulier un fil de guidage (41).

10. Dispositif médical selon au moins une des revendications 1 à 9,
**caractérisé en ce que**
l'élément de recanalisation (10) présente un revêtement libérant un principe actif, en particulier anti-thrombogène.

11. Dispositif médical selon au moins une des revendications 1 à 10,
**caractérisé en ce que**
l'élément de recanalisation (10), en particulier le corps creux (11), présente du moins par endroits un recouvrement étanche au fluide (12a) qui comprend en particulier un ou plusieurs orifices d'aspiration (12b) qui sont tournés à l'usage vers une concrétion (30).

12. Dispositif médical selon au moins une des revendications 1 à 11,
**caractérisé en ce que**
l'élément de recanalisation (10) est raccordé à une extrémité distale (14) à un élément d'actionnement (19) qui s'étend sensiblement axialement en longueur le long de l'élément de recanalisation (10).

13. Dispositif médical selon la revendication 12,
**caractérisé en ce que**
l'élément d'actionnement (19) est orienté du moins partiellement le long d'un contour extérieur et/ou d'un contour intérieur de l'élément de recanalisation (10).

14. Ensemble comprenant un dispositif médical selon la revendication 1 et un système d'acheminement (40) pour un tel dispositif.

15. Procédé de fabrication d'un dispositif médical selon une des revendications précédentes, dans lequel un corps creux expansible et compressible (11) en matériau à mémoire de forme est glissé sur un mandrin en forme de spirale et traité thermiquement, de sorte qu'une forme de spirale est impartie au corps creux (11).
